# EUROPEAN PATENT APPLICATION

(11) **EP 4 389 877 A1**
(43) Date of publication of application: **26.06.2024**
(21) Application number: 22851828.8
(22) Date of filing: 11.07.2022
(51) Int. Cl.: C12N 5/0783, C12N 5/0789

(54) **RIC CELL AND PREPARATION METHOD THEREFOR AND USE THEREOF**

(30) Priority: 06.08.2021 CN 202110903886
(71) Applicant: Institute Of Zoology, Chinese Academy Of Sciences, Beijing 100101 (CN); Beijing Institute for Stem Cell and Regenerative Medicine, Beijing 100101 (CN)
(72) Inventor: WANG, Jinyong, Beijing 100101 (CN); HUANG, Dehao, Beijing 100101 (CN); LI, Jianhuan, Meizhou City, Guangdong 514400 (CN); HU, Fangxiao, Beijing 100101 (CN); WANG, Tongjie, Beijing 100101 (CN); XIA, Chengxiang, Beijing 100101 (CN); HU, Baoyang, Beijing 100101 (CN); HAO, Jie, Beijing 100101 (CN); WANG, Lei, Beijing 100101 (CN); WANG, Liu, Beijing 100101 (CN)
(74) Representative: Held, Stephan
(86) International application number: PCT/CN2022/104873
(87) International publication number: WO 2023/011114

(57) **Abstract**

Provided are a RIC cell, and a preparation method therefor and application thereof. The present invention particularly relates to the lineage-specific differentiation of a pluripotent or multipotent stem cell, specifically to a RIC cell differentiated from a stem cell, and a preparation method therefor and application thereof.

## Description

The present application is based on the application with the CN application number of 202110903886.0 and the filing date of August 6, 2021, and claims its priority. The disclosure content of the CN application is hereby incorporated into the present application in its entirety.

### Technical Field

The present application relates to the field of stem cell biology, especially to a RIC cell and preparation method therefor and application thereof, particularly to the lineage-specific differentiation of pluripotent or multipotent stem cells, specifically to a RIC cell differentiated from stem cell and its preparation method and application.

### Background Art

Pluripotent stem cells can differentiate into most cell types, including all derivative cells of ectoderm, endoderm and mesoderm. The lymphopoietic process starting from hematopoietic cell populations isolated from mouse or human bone marrow or human umbilical cord blood (UCB) or peripheral blood has been reported. However, little is known about the *in vitro* differentiation of pluripotent stem cells into lymphocytes, and the technology is not yet mature. At the same time, although innate immune cells derived from the lymphoid lineage have been used for tumor treatment in cancer patients in recent years, efficient production of such cells remains a huge challenge.

### Contents of the present invention

### Definition of Terms

In the context, unless otherwise stated, scientific and technical terms used have the meaning commonly understood by those skilled in the art. Moreover, the operating procedures such as molecular genetics, nucleic acid chemistry, cell culture, biochemistry, and cell biology used herein are routine procedures widely used in the corresponding fields. Meanwhile, in order to better understand the present invention, definitions and explanations of relevant terms are provided below.

As used herein, the term "embryonic stem cells" may refer to pluripotent stem cells isolated from embryos and maintained in *in vitro* cell culture. These cells are cultured cells that are isolated from cultured embryos, divide rapidly and maintain pluripotency. Embryonic stem cells can be cultured with or without feeder cells. Embryonic stem cells can be isolated from early embryos, including but not limited to blastocyst-stage embryos and pre-blastocyst-stage embryos. Embryonic stem cells can have round cell morphology and can grow as round cell clumps on feeder layers. Embryonic stem cells are well known to those skilled in the art. Their sources may be of human or non-human animals. Human embryonic stem cells are limited to stem cells isolated from human embryos within 14 days of fertilization that have not undergone *in vivo* development.

As used herein, the term *"in vitro"* refers to an artificial environment, and processes and reactions therein. *In vitro* environments are exemplified by test tubes and cell cultures, but are not limited thereto.

As used herein, the term *"in vivo"* refers to a natural environment (i.e., an animal or cell) and processes and reactions therein.

As used herein, the term "culture" refers to a product obtained by culturing cells (e.g., the population of RIC cells of the present invention) in a culture medium.

As used herein, the term "culture supernatant" refers to a culture fluid that is obtained by culturing cells (e.g., the population of RIC cells of the present invention) and does not contain the cells *per se.* Therefore, for example, a culture supernatant usable in the present invention can be obtained by separating and removing cellular components after culture. The culture supernatant can also undergo other treatments, such as centrifugation, concentration, solvent replacement, dialysis, freezing, drying, freeze-drying, dilution, desalting, storage, etc.

As used herein, the term "pharmaceutically acceptable carrier or excipient" refers to a carrier and/or excipient that is pharmacologically and/or physiologically compatible with the subject and active ingredients, and is well known in the art (see, for example, Remington's Pharmaceutical Sciences. Edited by Gennaro AR, 19th ed. Pennsylvania: Mack Publishing Company, 1995), and includes, but is not limited to: pH adjusters, surfactants, ionic strength enhancers, reagents for maintaining osmosis pressure, reagents for delaying absorption, diluents, adjuvants, preservatives, etc. For example, the pH adjuster includes, but is not limited to, phosphate buffer. The surfactant includes, but is not limited to, cationic, anionic or nonionic surfactant, such as Tween-80. The ionic strength enhancer includes, but is not limited to, sodium chloride. The reagent for maintaining osmotic pressure includes, but is not limited to, sugar, NaCl, and the like. The reagent for delaying absorption includes, but is not limited to, monostearate and gelatin. The diluent includes, but is not limited to, water, aqueous buffers (e.g., buffered saline), alcohols and polyols (e.g., glycerol), and the like. The adjuvant includes, but is not limited to, aluminum adjuvant (e.g., aluminum hydroxide), Freund's adjuvant (e.g., complete Freund's adjuvant), and the like. The preservative includes, but is not limited to, various antibacterial and antifungal agents, such as thimerosal, 2-phenoxyethanol, paraben, chlorobutanol, phenol, sorbic acid, etc. In certain embodiments, the pharmaceutically acceptable carrier or excipient is a sterile isotonic aqueous or non-aqueous solution (e.g., balanced salt solution or physiological saline), dispersion, suspension, or emulsion.

As used herein, the term "prevention" refers to a method performed to prevent or delay the onset of a disease or disorder or symptom in a subject or to minimize the effect if it occurs. The term "treatment" refers to a method performed to obtain a beneficial or desired clinical result. Beneficial or desired clinical result includes, but is not limited to, reduced rate of disease progression, improvement or alleviation of disease status, and regression or improved prognosis, whether detectable or undetectable. The amount of a therapeutic agent effective in alleviating the symptom of any particular disease can vary depending on factors such as the disease state, age and weight of the patient, and the ability of drug to elicit a desired response in the subject. The relief of disease symptoms may be assessed by any clinical measures, and these measures are typically used by a physician or other skilled health care provider to assess the severity or progression of the symptoms.

As used herein, the term "effective amount" refers to an amount sufficient to obtain, at least in part, a desired effect. For example, an effective amount for preventing a disease refers to an amount sufficient to prevent, arrest, or delay the occurrence of the disease; an effective amount for treating a disease refers to an amount sufficient to cure or at least partially arrest the disease and its complications in a patient who already suffers from the disease. Determining such effective amounts is well within the capabilities of those skilled in the art. For example, the effective amount for therapeutic use will depend on the severity of the disease to be treated, the overall status of the patient's own immune system, the patient's general condition such as age, weight and gender, the route of the administration of the drug, and other treatments administered concurrently, etc.

As used herein, the term "subject" includes, but is not limited to, various animals, such as mammals, such as bovine, equine, ovine, porcine, canine, feline, leporid, rodent (e.g., mouse or rat), non-human primate (e.g., macaque or cynomolgus monkey), or human.

As used herein, the term "proliferation" or "expansion" refers to the ability of a cell or population of cells to increase in number.

As used herein, the term composition containing a "purified cell population" or "purified cell composition" means that at least 30%, 50%, 60%, usually at least 70%, and more preferably 80%, 90% %, 95%, 98%, 99% or more of the cells in the composition are of the identified type.

As used herein, the term "primary immune cells" refers to natural killer cells isolated directly from tissue taken from the body (e.g., cord blood, peripheral blood, or bone marrow).

As used herein, the term "CAR" refers to a chimeric antigen receptor.

As used herein, the term "expression rate" refers to a positive ratio of cells to express a specific protein. For example, "80% CAR expression rate" means that 80% of cells express CAR.

As used herein, the term "RIC capsule" refers to a capsule-like structure from which RIC cells are produced.

As used herein, the term "RIC cell" refers to an immune cell or cell population of the present invention, the characteristics of which are set forth in the description.

As used herein, the term "seed cell" refers to an immature cell capable of forming a mature cell.

As used herein, the term "X+ cell" refers to a cell that is X positive or expresses an X gene. For example, CD34+ cells are cells that express CD34. The expression of X can be detected using existing technology.

As used herein, the term "X- cell" refers to a cell that is X-negative or does not express an X gene. For example, CD34- cells are cells that do not express CD34. The expression of X can be detected using existing technology.

As used herein, the term "NKp30/NCR3" refers to a member of IgSF family, is a natural cytotoxic receptor distributed in resting and activated NK cells, and activates signals by transduction of ITAM-containing DAP12; and cooperates with NKp46 to mediate the killing effect of NK cells.

As used herein, the term "NKp44/NCR2" refers to a member of IgSF family, is a natural cytotoxic receptor expressed in activated NK cells, and participates the mediation of the killing activity of NK cells through ITAM-containing DAP12.

As used herein, the term "NKG2C/KLRC2" refers to a receptor capable of binding to the non-classical HLA class I molecule HLA-E, and transmit an activating signal. NKG2C is capable of recognizing a peptide of UL40 presented by HLA-E in cells infected with HCMV and promoting the release of IPNγ.

As used herein, the term "NKG2D/KLRK1" refers to an activating receptor expressed on human natural killer cells, and DAP10 binds only to the intracellular domain of NKG2D to pass the activation signal. It plays an important role in natural immunity and is involved in the recognition of virus-infected cells and the killing of tumor cells.

As used herein, the term "SLAMF7/CS1" refers to signaling lymphocyte activating molecule family member 7, which is expressed on the surface of natural killer cells. There are two variants of human CS1 molecules, namely long-form CS1-L and short-form CS1-S, both of which can be expressed on NK cells, in which CS1-L is an NK-activating receptor, while CS1-S cannot activate NK cells.

As used herein, the term "KLRB1/CD161" refers to killer cell lectin-like receptor subfamily B member 1, which belongs to NK cell inhibitory receptors.

As used herein, the term "KLRC1/NKG2A" refers to a receptor capable of binding to the non-classical HLA class I molecule HLA-E to deliver an inhibitory signal. Uterine NK cells highly express NKG2A. Under normal physiological conditions, placental trophoblast cells express HLA-E, which interacts with NKG2A, inhibits the killing effect of uterine demodulation NK cells, and maintains placental immune tolerance. However, under pathological conditions, tumor cells upregulate HLA-E molecules and can escape the killing by NK cells.

As used herein, the term "LAIR1" refers to leukocyte-associated immunoglobulin-like receptor 1, which belongs to NK cell inhibitory receptors.

As used herein, the term "SIGLEC7" refers to sialic acid-binding immunoglobulin-like lectin 7.

As used herein, the term "CD96" and TIGIT are NK cell inhibitory receptors, which can also bind to CD155 and CD112, wherein CD155 is the ligand for DNAM-1. These two inhibitory receptors and DNAM-1 form the TIGIT-CD226-CD96 receptor family.

As used herein, the term "NCAM1/CD56" refers to neural cell adhesion molecule 1, which is expressed primarily on NK or NKT cells among the immune cells.

As used herein, the term "KLRD1/CD94" refers to human killer cell lectin-like receptor D1. CD94 molecules can form dimers with NKG2A, recognize HLA-E molecules, and transmit inhibitory signals.

As used herein, the term "CD69" is a type II transmembrane protein and can be expressed on activated T cells and NK cells.

As used herein, the term "TNSFSF10/TRAIL" refers to human tumor necrosis factor-related apoptosis-inducing ligand, which participates the mediation of apoptosis.

As used herein, the term "FASLG" refers to Fas ligand, which participates the mediation of apoptosis.

As used herein, the term "PRF1/Perforin" refers to perforin, which can disrupt target cell membranes.

As used herein, the term "GZMA/Granzyme A" refers to granzyme A, which enters target cells through pores made by perforin and induces caspase-independent cell death.

As used in this article, the term "GZMB/Granzyme B" refers to granzyme B, which is the main effector molecule of granzymes. After entering target cells, it can activate the Caspase cascade reaction, thereby rapidly causing target cell apoptosis.

As used herein, the term "therapeutically effective" refers to an amount of RIC cells sufficient to treat or ameliorate or in some manner reduce symptoms associated with a disease, such as a cancer, or a condition, such as an infection-associated condition. When used with reference to a method, the method is effective enough to treat or ameliorate or in some way alleviate the symptoms associated with a disease or condition. For example, an effective amount for a disease is an amount sufficient to arrest or prevent the onset of the disease; or if the pathology of the disease has already begun, to alleviate, ameliorate, stabilize, reverse or slow down the progression of the disease, or otherwise lessen the pathological consequences of the disease. In any case, the effective amount may be achieved by administration of a single dose or divided doses.

As used herein, the term "pharmaceutical composition" refers to a pharmaceutically acceptable composition, wherein the composition comprises RIC cells, and in some embodiments further comprises a pharmaceutically acceptable carrier. In some embodiments, the pharmaceutical composition may be a combination.

As used herein, the term "combination" refers to a fixed combination existing in dosage unit form, or to a kit of parts for combined administration, in which RIC cells and a combination partner (e.g., another drug as explained below, also known as "therapeutic agent" or "co-agent") may be administered simultaneously or separately within a time interval. In some cases, the combination partner exhibits synergistic effect, such as synergy.

As used herein, the term "co-administration" or "combined administration" and the like are intended to encompass the administration of a selected combination partner to a single subject in need thereof, and are intended to include treatment regimens in which they are not necessarily administrated by the same route or at the same time.

As used herein, genome editing tools include, for example, clustered regularly interspaced short palindromic repeats (CRISPR) system that can be used in a variety of organisms (e.g., to add, disrupt, or alter DNA sequences of a specific gene), TALEN editing tools, etc. As used herein, the term "epitope" includes any protein determinant of antigen capable of specifically binding to an antibody or T cell receptor. Epitope determinant typically consists of chemically active molecular surface groups, such as amino acids or sugar side chains, and often has specific three-dimensional structural characteristics as well as specific charge characteristics.

As used herein, the term "antibody" encompasses monoclonal antibodies (including full-length monoclonal antibodies), polyclonal antibodies, multispecific antibodies (e.g., bispecific antibodies) and antibody fragments, so long as they exhibit the required biologically active ability to bind to target antigenic sites and congeners of interest. The term "antibody fragment" encompasses a portion of a full-length antibody, typically is its antigen-binding region or its variable region or constant region. As used herein, the term "antibody" encompasses any antibody derived from any species and source, including but not limited to human antibody, rat antibody, mouse antibody, rabbit antibody, etc., and may be synthetically prepared or naturally occurring.

As used herein, the term "differentiation" refers to a process by which less specialized cells become more specialized cells to form the progeny of at least one new cell type in culture or *in vivo,* rather than no differentiation under the same conditions. Under certain conditions, the proportion of progeny with characteristics of the new cell type can be at least about 1%, 5%, 25%, or more to increase the priority.

### Population of RIC cells

In a first aspect, the present invention provides a population of RIC cells, wherein the average expression level of NKG2E gene of the RIC cells (e.g., without genetic modification) is at least about 10 times (e.g., at least about 20 times, at least about 30 times, at least about 50 times, at least about 60 times, at least about 80 times, at least about 90 times, at least about 100 times, at least about 150 times, at least about 200 times, at least about 300 times, at least about 400 times, at least about 500 times) that of primary immune cells.

In certain embodiments, the expression level of SELL and CD2 of the population of RIC cells is at least about 10 times (e.g., at least about 20 times, at least about 30 times, at least about 40 times, at least about 50 times, at least about 60 times, at least about 70 times, at least about 80 times, at least about 90 times, at least about 100 times, at least about 150 times, at least about 200 times, at least about 300 times, at least about 400 times, at least about 500 times) less than that of primary immune cells.

In the population of RIC cells, the content of RIC cells is no less than 60%, no less than 70%, no less than 80%, no less than 90%, and no less than 95%.

In certain embodiments, the population of RIC cells has any three or more, or four, or all of the activating receptors NCR1, NCR2, NCR3, KLRC2, KLRK1 and SLAMF7; and has no significant difference compared with an equal number of primary immune cells. In certain embodiments, the population of RIC cells has any three or more, or four, or all of the inhibitory receptors KLRB1, KLRC1, LAIR1, SIGLEC7 and CD96, and has no significant difference compared with an equal number of primary immune cells.

In certain embodiments, the population of RIC cells has any three or more, or four, or all of the activating receptors NCR1, NCR2, CD96, KLRC2 and KLRK1; and has no significant difference compared with an equal number of primary immune cells. In certain embodiments, the population of RIC cells has any three or more, or four, or all of the inhibitory receptors KLRB 1, KLRC1, LAIR1 and SIGLEC7, and has no significant difference compared with an equal number of primary immune cells.

In certain embodiments, the population of RIC cells has any three or more, or four, or all of the receptors NCR1, NCR2, CD96, KLRC2 and KLRK1, and has no significant difference compared with an equal number of primary immune cells. In certain embodiments, the population of RIC cells has any three or more, or four, or all of the receptors KLRB1, KLRC1, LAIR1 and SIGLEC7, and has no significant difference compared with an equal number of primary immune cells.

In some embodiments, the population of RIC cells is introduced with a target CAR by a gene delivery vector or continuously or conditionally expresses a target CAR by gene editing, wherein the expression rate of CAR is 60% to 100%, preferably at least 70%, at least 80%, at least 90%, at least 95%. Optionally, the expression rate of CD19-targeting CAR is 60% to 100%, preferably at least 70%, at least 80%, at least 90%, or at least 95%.

As used herein, the expression may be monitored by measuring the level of full-length mRNA, mRNA fragment, full-length protein or protein fragment of the gene. Thus, in certain embodiments, the expression level is mRNA level or protein level.

In some embodiments, the expression is assessed by analyzing the expression of mRNA transcript of the gene. For example, the expression of the above genes in the cell population can be determined by measuring the presence or content of mRNA for *TBXT, APLNR, HAND1, CD2, SELL, NKG2-E, SLAMF7, NCR2, NCR3, CD96, KLRC2, KLRK1, KLRB1, KLRC1, EAIR1* or *SIGLEC7* by RT-PCR.

In other embodiments, the expression is assessed by analyzing the expression of a protein product of the gene. For example, the expression of the above gene in the cell population can be determined by measuring the expression of the protein BRACHYURY, APLNR, CD45, CD43, CD34 or CD56 on the cell membrane of the cell population by immunological detection.

In certain embodiments, the RIC cells possess one or more of the gene expression characteristics described above in the condition of not being genetically modified. The term "not being genetically modified" means that they have not undergone a process that an exogenous genetic material in form of DNA or RNA is introduced to the total genetic material of the cells, wherein the term "exogenous genetic material" can refer to an artificially introduced nucleotide sequence that is foreign to the cells not being genetically modified. It is easy to understand that the above term "not being genetically modified" is only used to describe the condition that the population of RIC cells of the present invention possesses one or more of the above gene expression characteristics, and is not used to limit that the population of RIC cells of the present invention cannot contain a genetic modification. Thus, in certain embodiments, the RIC cells of the present invention may comprise one or more genetic modifications.

In certain embodiments, the population of RIC cells is generated from stem cells. Preferably, the stem cells are totipotent stem cells or pluripotent stem cells, further preferably, the stem cells are pluripotent stem cells, further preferably, the pluripotent stem cells are selected from the group consisting of embryonic stem cells, haploid stem cells and induced pluripotent stem cells.

In certain embodiments, the population of RIC cells is generated *in vitro.*

In certain embodiments, ≥80% (e.g., ≥85%, ≥90%, ≥95%, ≥96%, ≥97%, ≥98%, ≥99%, or 100%) cells in the population of RIC cells express both CD45 and CD56.

The population of RIC cells of the present invention can be formulated and administered as a pharmaceutical composition. Such pharmaceutical composition can be in any form known in the medical field, preferably injection (including injection solution and freeze-dried powder). In certain embodiments, the pharmaceutical composition comprises a pharmaceutically acceptable sterile isotonic aqueous or non-aqueous solution (e.g., balanced salt solution or physiological saline), dispersion, suspension, or emulsion. General principles for formulating the pharmaceutical composition may be found in "Cell Therapy: Stem Cell Transplantation, Gene Therapy, and Cellular Immunotherapy", edited by G. Morstyn and W. Sheridan, Cambridge University Press, 1996; and "Hematopoietic Stem Cell Therapy", E. D. Ball, J. Lister & P. Law, Churchill Livingstone, 2000.

### Method for producing population of RIC cells

In a second aspect, the present invention provides a method for producing the population of RIC cells described in the first aspect, which comprises the following steps:
(1) culturing stem cells to form lateral plate mesoderm cells;
(2) inducing the lateral plate mesoderm cells to differentiate into hematopoietic progenitor cells;
(3) inducing the hematopoietic progenitor cells to differentiate into RIC cells.

More than 65% of the lateral plate mesoderm cells are APLNR+ cells; for example, more than 70% of the cells are APLNR+ cells; for example, more than 75% of the cells are APLNR+ cells; for example, more than 80% of the cells are APLNR+ cells; for example, more than 85% of the cells are APLNR+ cells; for example, more than 90% of the cells are APLNR+ cells; for example, more than 95% of the cells are APLNR+ cells.

More than 65% of the lateral plate mesoderm cells are APLNR+HAND1+ cells; for example, more than 70% of the cells are APLNR+HAND1+ cells; for example, more than 75% of the cells are APLNR+HAND1+ cells; for example, more than 80% of the cells are APLNR+HAND1+ cells; for example, more than 85% of the cells are APLNR+HAND1+ cells; for example, more than 90% of the cells are APLNR+HAND1+ cells; for example, more than 95% of the cells are APLNR+HAND1+ cells; for example, more than 99% of cells are APLNR+HAND1+ cells.

More than 65% of the lateral plate mesoderm cells are APLNR+HAND1+MSGN1-OSR1-cells; for example, more than 70% of the cells are APLNR+HAND1+MSGN1-OSR1- cells; for example, more than 75% of the cells are APLNR+HAND1+MSGN1-OSR1- cells; for example, more than 80% of the cells are APLNR+HAND1+MSGN1-OSR1- cells; for example, more than 85% of the cells are APLNR+HAND1+MSGN1-OSR1- cells; for example, more than 90% of the cells are APLNR+HAND1+MSGN1-OSR1- cells; for example, more than 95% of the cells are APLNR+HAND1+MSGN1-OSR1- cells; for example, more than 99% of the cells are APLNR+HAND1+MSGN1-OSR1- cells.

More than 65% of the hematopoietic progenitor cells are CD45+CD43+CD34+CD235a-cells; for example, more than 70% of the cells are CD45+CD34+CD43+CD235a- cells; for example, more than 75% of the cells are CD45+CD43+CD34+CD235a- cells; for example, more than 80% of the cells are CD45+CD43+CD34+CD235a- cells; for example, more than 85% of the cells are CD45+CD43+CD34+CD235a- cells; for example, more than 90% of the cells are CD45+CD43+CD34+CD235a- cells; for example, more than 95% of the cells are CD45+CD43+CD34+CD235a- cells.

RIC cells can be obtained by induction in Step (3). A monolayer induction protocol can be adopted to induce and obtain high-purity and high-yield RIC cells, wherein RIC cells account for no less than 60%, no less than 70%, no less than 80%, no less than 90%, or no less than 95%.

During the process for producing the population of RIC cells of the present invention, spin polymerization is not required, and spin embryoid or EB (embryoid body) is not produced.

In the generalized overall scheme, step (1) of the present invention is to differentiate pluripotent stem cells into lateral plate mesoderm cells (LM) through monolayer induction. Alternatively, two serum-free media I and II are used successively with or without matrix. This step takes 2 to 3 days. There is no need to perform spin polymerization in step (1) and no embryoid body (EB) is generated. Step (2) of the present invention is to directly mix the generated lateral plate mesoderm cells, without sorting or enrichment, with a trophoblast cell expressing a combination of specific genes to prepare a RIC capsule. The culture of RIC capsule has undergone gradual culture induction in two different media (III, IV) sequentially to differentiate into LM and hematopoietic progenitor cells correspondingly, in which the stage from LM to hematopoietic progenitor cells takes 10 to 18 days; and step (3) is a stage of inducing the hematopoietic progenitor cells to RIC (regenerative immune cells, RIC for short), and the development of RIC takes 7 to 14 days. Alternatively, RIC can also be obtained by removing the trophoblast cell at the stage of hematopoietic progenitor cells and then performing monolayer induction. The entire culture of RIC capsule takes 17 to 32 days in total.

In some embodiments, the lateral plate mesoderm cells of step (1) are APLNR+HAND1+ cells obtained by monolayer induction of pluripotent stem cells.

The above steps may adopt the following technical solutions:
(1) Media I and II are used to culture stem cells to form lateral plate mesoderm cells; wherein, Medium I is a basic medium obtained supplemented with the following substances: one or more serum substitutes, one or more non-essential amino acids, glutamine or a stabilized dipeptide of L-alanyl-L-glutamine, as well as BMP4, ACTIVIN A, bFGF, Wnt agonist (e.g., small molecular compound CHIR-99021) and PI3K inhibitor (e.g., small molecular compound PIK-90, LY294002); Medium II is a basic medium supplemented with the following substances: one or more serum substitutes, one or more non-essential amino acids, glutamine or a stabilized dipeptide of L-alanyl-L-glutamine, as well as BMP4, TGF-β inhibitor (e.g., small molecule compounds A-83-01, SB431542) and Wnt inhibitor (e.g., small molecule compounds C59, IWR-1- endo);
   In some embodiments, Medium I is a basic medium supplemented with one or more serum substitutes, one or more non-essential amino acids, glutamine or a stabilized dipeptide of L-alanyl-L-glutamine, as well as BMP4, ACTIVIN, bFGF, CHIR-99021 and PIK-90; Medium II is a basic medium supplemented with the following substances: one or more serum substitutes, one or more non-essential amino acids, glutamine or a stabilized dipeptide of L-alanyl-L-glutamine, as well as BMP4, A-83-01 and C59;
(2) Medium III is used to induce the differentiation of the lateral plate mesoderm cells into hematopoietic progenitor cells; wherein Medium III is a basic culture medium supplemented with the following substances: one or more serum substitutes, one or more non-essential amino acids, glutamine or a stabilized dipeptide of L-alanyl-L-glutamine, TGF-β inhibitor (e.g., small molecule compounds A-83-01, SB431542), hydrocortisone, ascorbic acid, and cell growth factor such as one, two or more selected from the group consisting of Flt3L, TPO, SCF, EGF, VEGF, bFGF and IGF-1; in some embodiments, Medium III is a basic medium supplemented with one or more serum substitutes, one or more non-essential amino acids, glutamine or a stabilized dipeptide of L-alanyl-L-glutamine, and cell growth factor such as one, two or more selected from the group consisting of Flt3L, TPO, SCF, EGF, VEGF, bFGF and IGF-1;
(3) Medium IV is used to induce the differentiation of the hematopoietic progenitor cells into RIC cells; wherein Medium IV is a basic culture medium supplemented with the following substances: one or more serum substitutes, one or more non-essential amino acids, glutamine or a stabilized dipeptide of L-alanyl-L-glutamine, sodium selenite, ethanolamine, ascorbic acid, and cell growth factor such as one, two or more selected from the group consisting of IL-3, SCF, IL-7, IL-15 and Flt3L. In certain embodiments, Medium IV further comprises β-mercaptoethanol. In certain embodiments, the content of β-mercaptoethanol is 0.1 to 0.5% (v/v), such as about 0.1% (v/v), about 0.2% (v/v), about 0.3% (v/v), about 0.4% (v/v), or about 0.5% (v/v);

In some embodiments, Medium IV is a basic medium supplemented with one or more serum substitutes, one or more non-essential amino acids, glutamine, stabilized dipeptide of L-alanyl-L-glutamine, as well as cell growth factor such as one, two or more selected from the group consisting of IL-3, SCF, IL-7, IL-15, and Flt3L; in certain embodiments, Medium IV further comprises β-mercaptoethanol; in certain embodiments, the content of β-mercaptoethanol is 0.1 to 0.5% (v/v), such as about 0.1% (v/v), about 0.2% (v/ v), about 0.3% (v/v), about 0.4% (v/v), or about 0.5% (v/v).

As used herein, the term "basic medium" refers to any medium capable of supporting cell growth, typically containing inorganic salts, vitamins, glucoses, buffer system and essential amino acids, and typically having an osmotic pressure of approximately 280 to 330 mOsmol. For example, it is selected from the group consisting of Essential 6, DMEM-high glucose, DMEM/F12, α-MEM, F-12, EBM2, MEM, BME, RPMI 1640, G-MEM, and any combination thereof.

In some embodiments, the stem cells described in step (1) are totipotent stem cells or pluripotent stem cells, further preferably, the stem cells are pluripotent stem cells, further preferably, the pluripotent stem cells are selected from embryonic stem cells, haploid stem cells, induced pluripotent stem cells.

In certain embodiments, the culturing described in step (1) is performed in a matrix. In certain embodiments, the matrix comprises an extracellular matrix. In certain embodiments, the matrix comprises one or more selected from the group consisting of Growth Factor Reduced Matrigel (GR-MTG material), glycoprotein (for example, but not limited to vitronectin (VTN), fibronectin), hyaluronic acid, laminin, fibronectin, collagen, elastin, heparan sulfate, dextran, dextran sulfate, and chondroitin sulfate. Preferably, the matrix is a glycoprotein. Preferably, the matrix is vitronectin (VTN) and/or fibronectin. Preferably, the matrix is vitronectin (VTN).

In certain embodiments, the cells generated in step (2) comprise: lateral plate mesoderm cells, hematopoietic endothelial cells, hematopoietic stem/progenitor cells, and immune progenitor cells; step (2) comprises: the RIC capsule and media III, IV, preferably Medium III, are used to perform the culturing. Preferably, the RIC capsule is attached to a culture vessel.

In certain embodiments, Media I, II, III, IV possess one or more of the following characteristics:
(i) the total content of the one or more serum substitutes is 2% to 30% (v/v), such as 3% to 30% (v/v), about 2% (v/v), about 3% (v/v), about 5% (v/v), about 8% (v/v), about 10% (v/v), about 12% (v/v), about 15% (v/v), about 18% (v/v), about 20% (v/v), about 22% (v/v), about 25% (v/v), about 28% (v/v), or about 30% (v/v);
(ii) the content of each of the one or more non-essential amino acids is 0.1 to 0.5 mM, such as about 0.1 mM, about 0.2 mM, about 0.3 mM, about 0.4 mM or about 0.5 mM;
(iii) the content of glutamine or the stabilized dipeptide of L-alanyl-L-glutamine is 1 to 5 mM, such as about 1 mM, about 2mM, about 3mM, about 4mM, or about 5mM;
(iv) the content of each of the factors of BMP4, ACTIVIN, bFGF, Flt3L, TPO, SCF, EGF, VEGF, bFGF, IGF-1, IL-3, IL-7 and IL-15 is independently 1 to 100 ng/mL, such as 2 to 100 ng/mL, 2 to 50 ng/mL, 5 to 100 ng/mL, 5 to 50 ng/mL, or 5 to 20 ng/mL; such as about 1 ng/mL, about 2 ng/mL, about 3 ng/mL, about 5 ng/mL, about 8 ng/mL, about 10 ng/mL, about 15 ng/mL, about 20 ng/mL, about 25 ng/mL, about 30 ng/mL, about 35 ng/mL, about 40 ng/mL, about 45 ng/mL, about 50 ng/mL, about 55 ng/mL, about 60 ng/mL, about 65 ng/mL, about 70 ng/mL, about 75 ng /mL, about 80 ng/mL, about 85 ng/mL, about 90 ng/mL, about 95 ng/mL, or about 100 ng/mL.
(v) the content of each of the small molecules of Wnt agonist, PI3K inhibitor, TGF-β inhibitor and Wnt inhibitor may be 10nM to 100µM, such as 10nM to 30nM, 10nM to 50nM, 10nM to 80nM, 10nM to 100nM, 100nM to 1µM, 1µM to 10µM, 10µM to 30µM, 10µM to 50µM, 10µM to 80µM, 30µM to 100µM.

In certain embodiments, Media I, II, III, IV possess one or more of the following characteristics:
(a) the serum substitutes are serum-free additives, specifically selected from the group consisting of KOSR, B27, Ultroser^{™} G, SUPERGROW and any combination thereof; preferably, the serum substitutes are SUPERGROW (Dakewe, 6122011) (hereinafter referred to as SUPERGROW), B27 (Gibco 17504-044);
(b) the non-essential amino acids are selected from the group consisting of glycine, L-alanine, L-asparagine, L-aspartic acid, L-glutamic acid, L-proline, L-serine and any combination thereof;
(c) the basic medium is selected from the group consiting of Essential 6, DMEM-high glucose, DMEM/F12, α-MEM, F-12, EBM2, MEM, BME, RPMI 1640, G-MEM and any combination thereof.

In certain embodiments, Media I, II, III, IV possess one or more of the following characteristics:
Medium I: Essential 6 medium containing BMP4, ACTIVIN A, bFGF, CHIR-99021 and PIK-90.
Medium II: Essential 6 medium containing BMP4, A-83-01 and C59.
Medium III: Essential 6 medium containing SB431542, hydrocortisone, Flt3L, TPO, SCF, EGF, VEGF, bFGF, IGF-1, ascorbic acid, and SUPERGROW cell culture supplement (Dakewe, 6122011).
Medium IV: DMEM-high glucose, DMEM/F12, SUPERGROW cell culture supplement (Dakewe, 6122011), GlutaMAX^{™}, β-mercaptoethanol, sodium selenite, ethanolamine, ascorbic acid, penicillin-streptomycin solution, IL-3, SCF, IL-7, IL-15 and Flt3L.

In certain embodiments, Medium I is Essential 6 medium containing 40 ng/mL BMP4, 30 ng/mL ACTIVIN A, 20 ng/mL bFGF, 6 µM CHIR-99021, and 100 nM PIK-90.

In certain embodiments, Medium II is Essential 6 medium containing 30 ng/mL BMP4, 1 µM A-83-01, and 1 µM C59.

In certain embodiments, Medium III is Essential 6 medium containing 10 µM SB431542, 10 µM hydrocortisone, 5 ng/mL Flt3L, 5 ng/mL TPO, 50 ng/mL SCF, 50 ng/mL EGF, 50 ng/mL VEGF, 50 ng/mL bFGF, 50 ng/mL IGF-1, 50 µg/mL ascorbic acid, and 2% SUPERGROW Cell Culture Supplement (Dakewe, 6122011).

In certain embodiments, Medium IV contains: 55% DMEM-high glucose, 30% DMEM/F12, 15% SUPERGROW Cell Culture Supplement (Dakewe, 6122011), 2 mM GlutaMAX^{™}, 1 µM β-mercapto Ethanol, 5 ng/mL sodium selenite, 50 uM ethanolamine, 20 µg/mL ascorbic acid, 1% penicillin-streptomycin solution, 5 ng/mL IL-3, 20 ng/mL SCF, 20 ng/mL IL-7, 10 ng/mL IL-15, and 10 ng/mL Flt3L.

In the context, the term "cell culture dish" refers to a culture dish with a coating on its surface, in which the coating can prevent protein adsorption on the surface of the culture dish, thus minimizing the adhesion of monolayer cells to the culture vessel. Such cell culture dishes are well known to those skilled in the art and include, but are not limited to, low attachment culture dishes of Corning (Cat. No. 3262).

In certain embodiments, the culturing in step (1) is performed for 1 to 3 days, such as about 1 day, about 1.5 days, about 2 days, about 2.5 days, about 3 days.

In certain embodiments, the culturing in step (2) is performed for 10 to 18 days, such as about 11 days, about 12 days, about 13 days, about 14 days, about 15 days, about 16 days, about 17 days.

In certain embodiments, the culturing in step (3) is performed for 7 to 14 days, such as about 8 days, about 9 days, about 10 days, about 11 days, about 12 days, about 13 days, about 14 days.

In some embodiments, the step (2) comprises inoculating the RIC capsule composed of the lateral plate mesoderm cells derived from step (1) and trophoblast cells to the culture vessel at a planar density of about 1 RIC capsule/1-10 cm², wherein the lateral plate mesoderm cells are not necessarily sorted or enriched. In certain embodiments, the total cell load of a single RIC capsule is in the range of 200,000 to 10,000,000 cells. In certain embodiments, the mixing ratio of the lateral plate mesoderm cells and trophoblast cells is in the range of 1:5 to 1:200. In certain embodiments, the trophoblast cells of the RIC capsule are one or more selected from the group consisting of AFT024, OP9, MS5, HS-5 or other stromal cells, which preferably express at least one, two, three, more or all of the following genes: DLL1, DLL4, IL7, IL15, IL3 and Flt3L. Further preferably, the trophoblast cells of the RIC capsule are OP9-DLL1, OP9-DLL4, or OP9-DLL1-DLL4.

In certain embodiments, the culturing in steps (2) and (3) is performed for 17 to 32 days, such as about 17 days, about 18 days, about 19 days, about 20 days, about 21 days, about 22 days, about 23 days, about 24 days, about 25 days, about 26 days, about 27 days, about 28 days, about 29 days, about 30 days, about 31 days, or about 32 days.

In certain embodiments, the steps (2) and (3) comprise replacing with fresh culture medium every day or every 1 to 7 days (e.g., every 1, 2, 3, 4, 5, 6 or 7 days).

The entire culture process from stem cells to RIC cells takes 19 to 34 days, such as about 19 days, about 20 days, about 21 days, about 22 days, about 23 days, about 24 days, about 25 days, about 26 days, about 27 days, about 28 days, about 29 days, about 30 days, about 31 days, about 32 days, about 33 days or about 34 days.

In certain embodiments, in step (1-3), the culture conditions are 37°C and 5% CO₂. In certain embodiments, in steps (1) to (3), the culture is performed in an incubator at 37°C and 5% CO₂.

In a third aspect, the present invention also relates to a population of RIC cells produced by the method of the second aspect.

In certain embodiments, the population of RIC cells is as defined in the first aspect.

In a fourth aspect, the present invention also relates to an intermediate cell combination, which comprises lateral plate mesoderm cells formed by culturing stem cells, such as lateral plate mesoderm cells produced in step (1) of the aforementioned method for producing population of RIC cells. In certain embodiments, more than 65% of the lateral plate mesoderm cells are APLNR+ cells, preferably more than 65% are APLNR+HAND1+ cells.

In a fifth aspect, the present invention also relates to an intermediate cell combination, which comprises hematopoietic progenitor cells produced in step (2) of the aforementioned method for producing the population of RIC cells, and other immune progenitor cells (e.g., myeloid cells). In certain preferred embodiments, more than 65% of the hematopoietic progenitor cells are CD45+CD43+CD34+CD235a- cells.

### RIC capsule

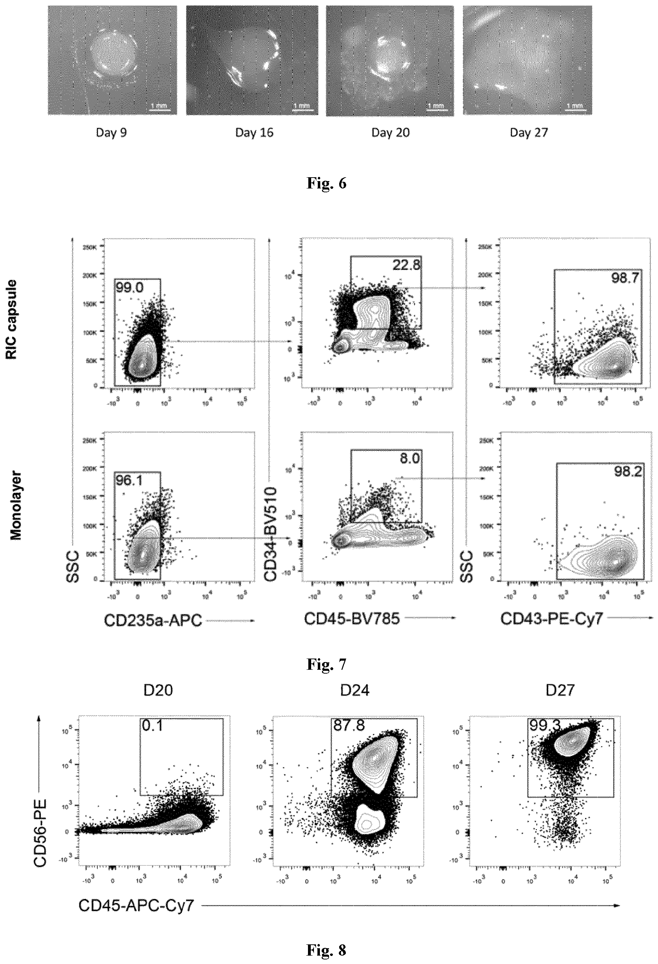

In a fifth aspect, the present invention also relates to a RIC capsule, which is composed of lateral plate mesoderm cells from previous step (1) and trophoblast cells. In some embodiments, the ratio of lateral plate mesoderm cells to trophoblast cells can be in the range of 1:5 to 1:200, such as 1:15, 1:10, 1:20, 1:30, 1:40,1:60, 1:90, 1:100, 1:110, 1:120, 1:130, 1:140, 1:150, 1:160, 1:170, 1:180, 1:190 or 1:200, etc. The trophoblast cells are preferably: AFT024, OP9, MS5, HS-5 or other stromal cells, which preferably express at least one, two, three, more or all of the following genes: DLL1, DLL4, IL7, IL15, IL3 and Flt3L. The synergistic effect of components of the RIC capsule greatly promotes the directional induction and differentiation of lateral plate mesoderm cells. The RIC capsule changes dynamically over time, and gradually changes from a smooth edge and uniform density state to a dispersed state, and expands from the center to the edge, which is specifically shown in Fig. 6. In some embodiments, one RIC capsule can be induced to obtain 1×10⁶, 2×10⁶, 3×10⁶, 4×10⁶, 5×10⁶ 6×10⁶, 7×10⁶, 8×10⁶, 9×10⁶ or 1×10⁷ RIC cells.

The total number of cells of a single RIC capsule may range from 200,000 to 10 million cells, such as 250,000, 300,000, 400,000, 600,000, 800,000, 1 million, 1.5 million, 1.8 million, 2 million, 3 million, 4 million, 5 million, 6 million, 7 million, 8 million, 9 million, etc. Each RIC capsule cultured occupies an area ranging from 1 to 10 cm². RIC capsule can be continuously cultured in Medium III for 10 to 18 days, and then in Medium IV for 7 to 14 days.

### Kit

In a sixth aspect, the present invention provides a kit, which comprises one, two, three or four of Media I, II, III, and IV provided separately.

The culture of the present invention can be formulated and administered as a pharmaceutical composition. Such pharmaceutical composition can be in any form known in the medical field, preferably injection (including injection solution and freeze-dried powder). In certain preferred embodiments, the pharmaceutical composition comprises a pharmaceutically acceptable sterile isotonic aqueous or non-aqueous solution (e.g., balanced salt solution or physiological saline), dispersion, suspension, or emulsion. General principles for formulating the pharmaceutical composition may be found in "Cell Therapy: Stem Cell Transplantation, Gene Therapy, and Cellular Immunotherapy" edited by G. Morstyn and W. Sheridan, Cambridge University Press, 1996; and "Hematopoietic Stem Cell Therapy", E. D. Ball, J. Lister & P. Law, Churchill Livingstone, 2000.

In the present invention, the population of RIC cells of the present invention can be cultured using any culture medium and culture conditions known in the art that can be used for immune cell culture.

### Use for prevention and/or treatment of disease

In a seventh aspect, the present invention relates to use of the population of RIC cells and/or the population of modified RIC cell-derived cells or the pharmaceutical composition containing the population of RIC cells and the population of derived cells thereof as described herein in a subject for preventing and/or treating a disease, or in the manufacture of a medicament for preventing and/or treating a disease in a subject, and a method of preventing and/or treating a disease in a subject, which comprises administering the population of RIC cells and/or the population of modified RIC cell-derived cells or the pharmaceutical composition containing the population of RIC cells and the population of derived cells thereof of the present invention to the subject in need thereof.

In certain embodiments, the subject is a mammal, such as a human.

In certain embodiments, the disease to be prevented and/or treated in the subject includes cancer, infectious disease, viral pneumonia, AIDS, immunodeficiency, immunocompromise or autoimmune disease.

In embodiments of each aspect of the present invention, examples of cancer include, but are not limited to, lymphoma, hematological malignancy, chronic or acute leukemia (including acute myeloid leukemia, chronic myeloid leukemia, acute lymphoblastic leukemia, chronic lymphocytic leukemia), multiple myeloma, lymphocytic lymphoma, breast cancer, ovarian cancer, liver cancer, glioma, pancreatic cancer, lung cancer, colon cancer, rectal cancer, melanoma, kidney cancer, bladder cancer, head and neck cancer, gastric cancer, nasopharyngeal cancer, laryngeal cancer, cervical cancer, uterine corpus tumor, osteosarcoma, bone cancer, skin cancer, prostate cancer, uterine cancer, anal area cancer, testicular cancer, fallopian tube cancer, endometrial cancer, vaginal cancer, vulva cancer, Hodgkin's disease, non-Hodgkin's lymphoma, esophageal cancer, small bowel cancer, endocrine system cancer, thyroid cancer, parathyroid cancer, adrenal gland cancer, soft tissue sarcoma, urethra cancer, penile cancer, pediatric solid tumor, bladder cancer, kidney or ureter cancer, renal pelvis cancer, central nervous system (CNS) tumor, primary CNS lymphoma, tumor angiogenesis, spinal tumor, brainstem glioma, pituitary adenoma, Kaposi's sarcoma, epidermal carcinoma, squamous cell carcinoma, T-cell lymphoma, environmentally induced cancer, including asbestos-induced cancer, and any combination thereof.

In embodiments of each aspect of the present invention, the infectious disease may be caused by a pathogen selected from viruses, bacteria, fungi, parasites or protozoa, including but not limited to human HIV virus, immunodeficiency virus, poliomyelitis virus, hepatitis A virus, enterovirus, rhinovirus, cytomegalovirus CMV, hepatitis B virus HBV, herpes simplex virus HSV, herpes zoster virus, coronavirus, rabies virus, Ebola virus, parainfluenza virus, mumps virus, measles virus, influenza virus, papilloma virus, polyoma virus; Pasteurella, Staphylococci, Streptococcus, Escherichia coli, Pseudomonas, Salmonella, Helicobacterpyloris, Mycobacteria, Neisseria gonorrhoeae, Neisseria meningitidis, Streptococcus, Erysipelothrixrhusiopathiae, Clostridium perfringers, Clostridium tetani, Enterobacter aerogenes, Klebsiellapneumoniae, Pasturella multocida, Leptospira and Rickettsia; Candida, Aspergillus, Cryptococcus, Histoplasma, Pneumocystis and Coccidioides; Babeosis bovis, Plasmodium, Leishmania spp., Toxoplasma gondii, and Trypanosoma cruzi.

In certain embodiments, the RIC cells are RIC cells containing a chimeric antigen receptor (CAR) (CAR-RIC cells).

In some embodiments, the CAR comprises an antigen-binding domain, such as an extracellular antigen-binding domain. In some embodiments, the antigen-binding domain is specific for an antigen of a pathogen that causes an infectious disease. In some embodiments, the antigen-binding domain is specific for a tumor associated antigen.

The tumor-associated antigen includes, but is not limited to, disialoganglioside GD2, epidermal growth factor receptor (EGFR), EGFR variant III (EGFRvIII), CD19, CD20, BCMA (CD269, TNFRSF17), NY-ESO-1, CD16, CD64, CD78, CD96, CLL1, CD116, CD117, CD71, CD45, CD71, CD123, CD138, ErbB2 (HER2/neu), carcinoembryonic antigen (CEA), epithelial cell adhesion molecule (EpCAM), CD30, CD40, duct epithelial mucin, gp36, TAG-72, glycosphingolipid, glioma-associated antigen, β-human chorionic gonadotropin, α-fetoglobin (AFP), lectin-responsive AFP, thyroglobulin, RAGE-1, MN-CA IX, human telomerase reverse transcriptase, RU1, RU2(AS), intestinal carboxyl esterase, mut hsp70-2, M-CSF, prostase, prostase-specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophilic granulocyte elastase, ephrin B2, CD22, insulin growth factor (IGF1)-I, IGF-II, IGFI receptor, mesothelin, major histocompatibility complex (MHC) presenting tumor-specific peptide epitope molecule, 5T4, ROR1, NKp30, tumor stromal antigen, extra domain A (EDA) and extra domain B (EDB) of fibronectin, tenascin-C A1 domain (TnC A1), fibroblast cell-associated protein (fap), CD3, CD4, CD8, CD24, CD25, CD33, CD34, CD133, CD138, Foxp3, B7-1(CD80), B7-2(CD86), GM-CSF, cytokine receptor, endothelial factor, major histocompatibility complex (MHC) molecule, TNFRSF17 (UNIPROT Q02223), SLAMF7 (UNIPROT Q9NQ25), GPRC5D (UNIPROT Q9NZD1), FKBP11 (UNIPROT Q9NYL4), KAMP3, ITGA8 (UNIPROT P53708), and FCRL5 (UNIPROT Q68SN8).

In embodiments of each aspect of the present invention, the antigen-binding domain may be, for example, a monoclonal antibody, a synthetic antibody, a human antibody, a humanized antibody, a single domain antibody, a nanobody, or an antibody single chain variable region (scFV), and antigen-binding fragment thereof. In some preferred embodiments, the antigen binding domain is a scFV.

In the present invention, the population of RIC cells and/or the modified RIC cell-derived cells or the pharmaceutical composition containing the population of RIC cells and the population of derived cells thereof, or the pharmaceutical composition comprising the population of RIC cells or culture as described herein can be administered to a subject in various suitable ways. In certain embodiments, the population of RIC cells and/or the modified RIC cell-derived cells or the pharmaceutical composition as described herein are administered to the subject via transplantation by local injection (e.g., stereotaxic intracerebral injection transplantation or spinal cord injection transplantation, injection into solid tumor), transplantation by blood circulation route (e.g., intravenous injection transplantation or intraarterial injection transplantation), or transplantation by cerebrospinal fluid route (e.g., lumbar puncture subarachnoid injection transplantation) or other routes. Those skilled in the art know how to select an appropriate cell transplantation route based on the location and nature of the lesion.

In certain embodiments, the medicament is in unit dosage form, and the unit dose of the medicament contains the population of RIC cells and/or the population of modified RIC cell-derived cells in a number of not less than 1×10⁴/ml (e.g., not less than 1×10⁴/ml, not less than 3×10⁴/ml, not less than 5×10⁴/ml not less than 7×10⁴/ml, not less than 1×10⁵/ml, not less than 3×10⁵/ml, not less than 5×10⁴/ml not less than 7×10⁵/ml, not less than 1×10⁶/ml, not less than 3×10⁶/ml, not less than 5×10⁶/ml, not less than 7×10⁶/ml, not less than 1×10⁷/ml, not less than 3×10⁷/ml, not less than 5×10⁴/ml not less than 7×10⁷/ml, not less than 1×10⁸/ml, not less than 3×10⁸/ml, not less than 5×10⁸/ml, not less than 7×10⁸/ml, not less than 1×10⁹/ml, not less than 3×10⁹/ml, not less than 5×10⁹/ml, not less than 7×10⁹/ml, not less than 1×10¹⁰/ml, not less than 3×10¹⁰/ml, not less than 5×10¹⁰/ml, or not less than 7×10¹⁰/ml). In some embodiments, the unit dose of the medicament contains the population of RIC cells and/or the population of modified RIC cell-derived cells in a number of 1×10⁴ to 1×10¹⁰ (e.g., 1×10⁶ to 1×10⁸, 1×10⁶ to 1×10⁷, or 1×10⁶ to 5×10⁶).

### Brief Description of the Drawings

The drawings described herein are used to provide a further understanding of the present invention and constitute a part of the present application. The illustrative examples of the present invention and their descriptions are used to explain the present invention and do not constitute an improper limitation of the present invention. In the drawings:
Fig. 1 shows a flow chart of induced differentiation of PSC into RIC, in which PSC includes ESC and iPSC; PS, primitive streak; LM, lateral plate mesoderm; hematopoietic progenitor cells, hematopoietic progenitor cells; RIC, inducible RIC cells;
Fig. 2 shows the cell morphology at different stage nodes of differentiation of embryonic stem cell in Example 1;

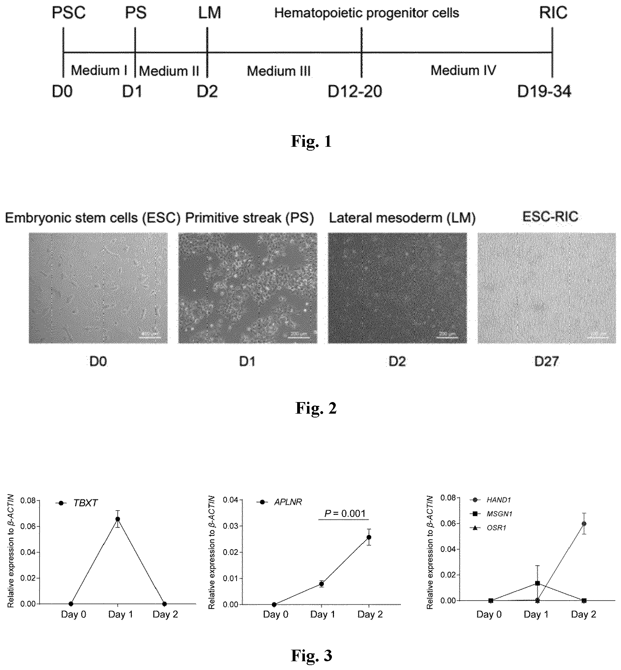

Fig. 3 shows the differentiation of embryonic stem cells in Example 1 into primitive streak *(TBXT+)* and lateral plate mesoderm cells (*APLNR+, HAND1*+)*.* The Y-axis value shows the result of qPCR analysis of differences in mRNA expression of related genes. The internal reference gene is *ACTIN,* and the target genes are *TBXT, APLNR, HAND1, MSGN1* and *OSR1. TBXT* is a primitive streak-specific marker gene, *APLNR* and *HAND1* are lateral plate mesoderm cell-specific marker genes, *MSGN1* is a paraxial mesoderm-specific marker gene, and *OSR1* is an intermediate mesoderm-specific marker gene. The analysis method is the 2^{-ΔCt} method, that is the relative expression of the target gene relative to the internal reference gene;
Fig. 4 shows the flow cytometry analysis of marker proteins of primitive streak (BRACHYURY+) and lateral plate mesoderm (APLNR+) in Example 1. BRACHYURY is a protein encoded by the TBXT gene and is an identity marker of primitive streak. APLNR is an identity marker of lateral plate mesoderm;
Fig. 5 shows a flow cytometry analysis diagram in Example 1. The flow cytometry analysis shows that, compared with differentiation using GR-MTG, the expression intensity of APLNR of ESC-derived lateral plate mesoderm differentiated using VTN was higher. VTN is vitronectin, GR-MTG is growth factor reduced matrigel;
Fig. 6 shows the RIC capsule morphology at different stage nodes of pluripotent stem cell differentiation in Example 2, which was taken with a stereoscope;
Fig. 7 shows that RIC capsules could effectively produce CD45+CD34+CD43+CD235a-hematopoietic progenitor cells;
Fig. 8 shows the changes of RIC in terms of maturity on Day 20, Day 24 and Day 27 in Example 3;
Fig. 9 shows the changes in the number of RIC cells on Day 20, Day 24 and Day 27;
Fig. 10 shows that, on D27, GFP+ trophoblast cells disappeared and RIC cells showed high purity;
Fig. 11 shows the yield statistics of RIC cells (D27) obtained by monolayer induction of hematopoietic progenitor cells isolated from RIC capsules (D20);
Fig. 12 shows the mature RIC cells obtained by induced regeneration in recipient mice transplanted with RIC capsule hematopoietic progenitor cells, wherein, BM refers to bone marrow, PB refers to peripheral blood, and SP refers to spleen;
Fig. 13 shows that ESC-RIC expresses activating receptors, wherein genes *NCR2, NCR3, KLRC2, KLRK1* and *SLAMF7* respectively encode the activating receptor protein NKp44, NKp30, NKG2C, NKG2D and CS1;
Fig. 14 shows that RIC cells express inhibitory receptors, wherein genes *KLRB1, KLRC1*, *LAIR1, SIGLEC7* and *CD96* respectively encode the inhibitory receptor protein CD161, NKG2A, LAIR-1, SIGLEC7 and CD96;
Fig. 15 shows that genes *NCAM1* and *KLRD1* respectively encode the protein CD56 and CD94, both of which were mainly related to the maturation of RIC cells; *CD69, TNFSF10, FASLG, PRF1, GZMA* and *GZMB* genes respectively encode CD69, TRAIL, FasL, Perforin, Granzyme A and Granzyme B, all of which were related to the activation and natural killing function of RIC cells;
Fig. 16 shows the unique expression gene pattern of RIC (derived from ESC) compared with primary immune cells. RIC highly expresses NKG2-E, and the expression level could reach more than 400 times that of the primary immune cells; it lowly expresses SELL and CD2, and the expression level was less than 1/50 of that of primary immune cells;
Fig. 17 shows the *in vitro* killing of tumor cells derived from lymphoma cell line Nalm-6 by RIC cells derived from pluripotent stem cells (ESC). ESC-1,-2,-3,-4,-5: five strains of ESC cell clones derived from National Stem Cell Resource Center. X-axis, E:T ratio (RIC effector cells: tumor cells). Y-axis, percentage of residual tumor cells. The absolute count of viable cells was detected after 6 hours of incubation;
Fig. 18 shows that ESC-RIC cells kill AML cells THP-1 and Molm13 efficiently *in vitro.* X-axis, E:T ratio (RIC effector cells: tumor cells). Y-axis, tumor cells killing percentage. Detection was conducted after 4 hours of co-incubation.
Fig. 19 shows that RIC cells derived from pluripotent stem cells (derived from ESC) killed tumor cells derived from ovarian cancer cell line A1847 *in vitro.* ESC-1,-2,-3,-4,-5: five strains of ESC cell clones derived from National Stem Cell Resource Center. X-axis, E:T ratio (RIC cells: tumor cells). Y-axis, percentage of the residual tumor cells accounting for the initially input tumor cells. Detection was conducted after 4 hours of co-incubation;
Fig. 20 shows that the killing of B lymphoma cell line Raji by RIC cells derived from pluripotent stem cells (ESC) is enhanced through ADCC effect. Rituximab is anti-CD20 rituximab. X-axis, E:T ratio (RIC cells: tumor cells). Y-axis, tumor cells killing percentage. Detection was conducted after 4 hours of co-incubation.
Fig. 21 shows the *in vivo* killing of A1847 ovarian cancer tumor cells by RIC (derived from ESC). a: Treatment flow and detection diagram; b to c: Dynamic *in vivo* imaging detection of tumor burden in the RIC treatment group and the tumor burden control group. *n*=5, statistical methods: one-way ANOVA and Kruskal-Wallis test. NS, not significant. d: Survival curve analysis. *n*=5, statistical method: Log-rank test. e: Body weight of tumor-bearing mice in the treatment group and the control group on Day 0 and Day 35. *n*=5, statistical method: two-sided independent *t*-test.

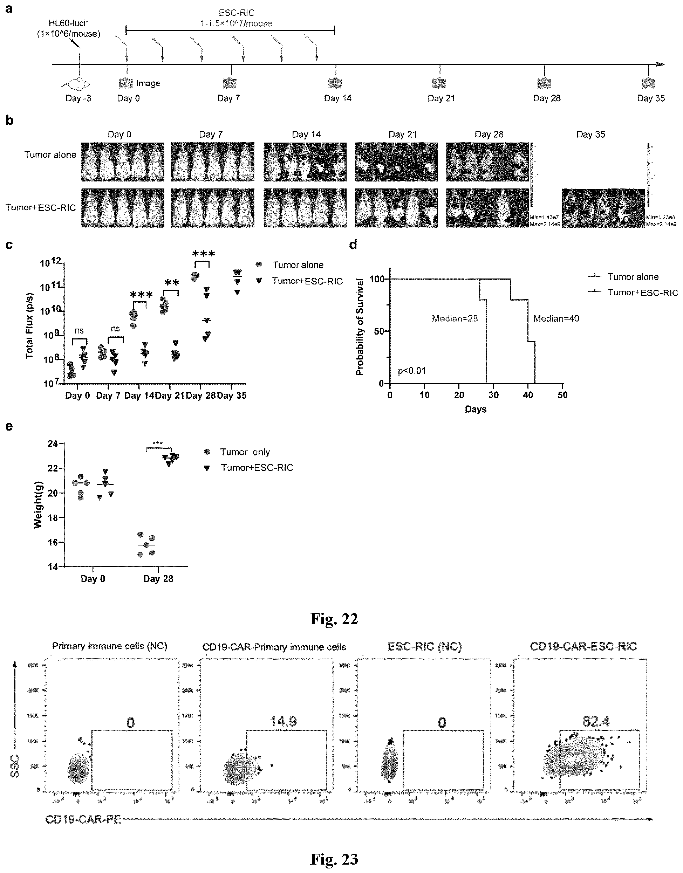

Fig. 22 shows the *in vivo* killing of HL60 acute myeloid leukemia cells by RIC (derived from ESC). a: Treatment flow and detection diagram. b to c: Dynamic *in vivo* imaging detection of tumor burden in the RIC treatment group and the tumor burden control group. *n*=5, statistical method: two-sided independent t-test. d: Survival curve analysis. *n*=5, statistical method: Log-rank test. e: Body weight of tumor-bearing mice in the treatment group and the control group on Day 0 and Day 28. n=5, statistical method: two-sided independent t-test. NS, not significant. *: p < 0.05, **: p < 0.01, ***: p < 0.001.
Fig. 23 shows the expression rate of CD19-CAR of RIC cells by infection. Compared with primary immune cells, the expression rate of CD19-CAR of RIC cells after infection with virus (MOI=20) carrying CD19-CAR was as high as 82.4%, whereas the expression rate of the natural immune cells (MOI=35) was only 14.9%.
Fig. 24 shows that CD19-CAR-ESC-RIC kills primary tumor cells of ALL acute lymphoblastic leukemia patients efficiently. The RIC cells carrying CD19-CAR were co-incubated *in vitro* with primary tumor cells (CD19+B cells) derived from two ALL patients at a ratio of 1:1 for 6 hours, and then the number of CD19+B cells was detected. Y-axis, percentage of residual tumor cells accounting for the initial input tumor;
Fig. 25 shows the cell morphology at different stage nodes of differentiation of induced pluripotent stem cell;

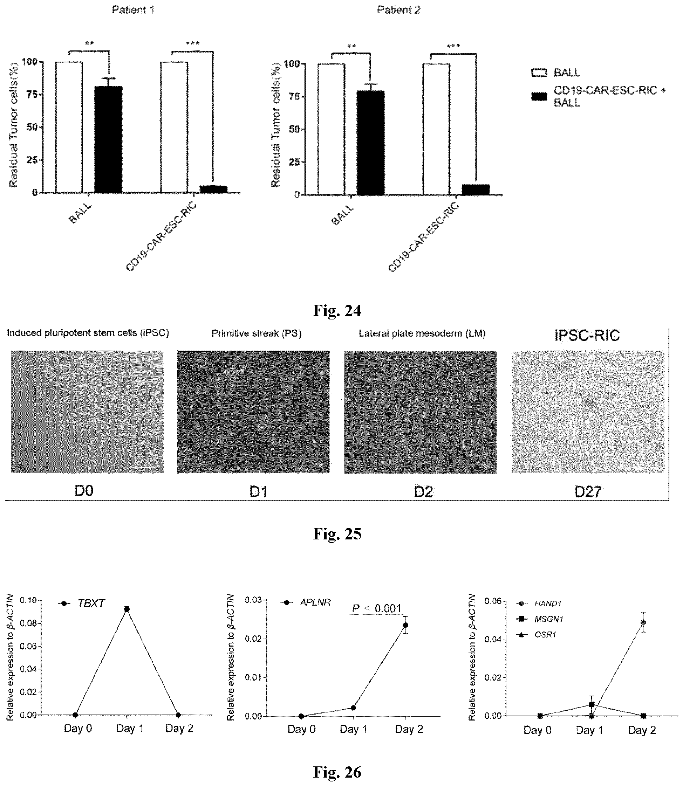

Fig. 26 shows the detection of specific cell markers for the differentiation of induced pluripotent stem cells into primitive streak *(TBXT+)* and lateral plate mesoderm cells (*APLNR+HAND1*+)*.* The Y-axis value is the result of qPCR analysis of differences in mRNA expression of related genes. The internal reference gene was ACTIN, and the target genes were *TBXT, APLNR, HAND1, MSGN1* and *OSR1. TBXT* is a primitive streak-specific marker gene, *APLNR* and *HAND1* are lateral plate mesoderm cell-specific marker genes, *MSGN1* is a paraxial mesoderm-specific marker gene, and *OSR1* is an intermediate mesoderm-specific marker gene. The analysis method was the 2^{-ΔCt} method, that was the relative expression of the target gene relative to the internal reference gene;
Fig. 27 shows the flow cytometry analysis of marker proteins of primitive streak (BRACHYURY+) and lateral plate mesoderm (APLNR+). BRACHYURY is a protein encoded by the *TBXT* gene and is a primitive streak identity marker. APLNR is a lateral plate mesoderm identity marker;
Fig. 28 shows the flow cytometry analysis, indicating that, compared with differentiation using GR-MTG material, the expression intensity of APLNR of iPSC-derived lateral plate mesoderm differentiated using VTN was higher. VTN is vitronectin, and GR-MTG is growth factor reduced matrigel;
Fig. 29 shows the changes of RIC in terms of maturity on Day 20, Day 24 and Day 27;
Fig. 30 shows the changes in the number of RIC cells on Day 20, Day 24 and Day 27;
Fig. 31 shows that, on D27, GFP+ trophoblast cells disappeared;
Fig. 32 shows the yield of RIC cells (D27) obtained by monolayer induction of hematopoietic progenitor cells isolated from RIC capsules (D20);
Fig. 33 shows that iPSC-RIC expresses activating receptors, wherein *NCR2, NCR3, KLRC2, KLRK1* and *SLAMF7* genes respectively encode the activating receptor protein NKp44, NKp30, NKG2C, NKG2D and CS1;
Fig. 34 shows that iPSC-RIC cells express inhibitory receptors, wherein *KLRB1, KLRC1*, *LAIR1, SIGLEC7* and *CD96* genes respectively encode the inhibitory receptor protein CD161, NKG2A, LAIR-1, SIGLEC7 and CD96;
Fig. 35 shows that genes NCAM1 and KLRD1 respectively encode the protein CD56 and CD94; *CD69, TNFSF10, FASLG, PRF1, GZMA* and *GZMB* genes respectively encode CD69, TRAIL, FasL, Perforin, Granzyme A and Granzyme B;
Fig. 36 shows the unique expression gene pattern of RIC (derived from iPSC) compared with primary immune cells. RIC highly expressed NKG2-E, and the expression level could reach more than 400 times that of the primary immune cells; and it lowly expressed SELL and CD2, and the expression level was less than 1/50 of that of the primary immune cells;
Fig. 37 shows the *in vitro* killing of tumor cells derived from lymphoma cell line Nalm-6 by RIC cells derived from pluripotent stem cells (iPSC). X-axis, E:T ratio (RIC effector cells: tumor cells). Y-axis, percentage of the residual tumor cells. Absolute count of viable cells was detected after 6 hours of co-incubation;
Fig. 38 shows the *in vitro* killing of tumor cells derived from ovarian cancer cell line A1847 by RIC cells derived from pluripotent stem cells (iPSC). X-axis, E:T ratio (RIC cells: tumor cells). Y-axis, percentage of the residual tumor cells accounting for the initial input tumor cells. Detection was conducted after 4 hours of co-incubation;
Fig. 39 shows that the killing of B lymphoma cell line Raji by RIC cells from pluripotent stem cells (iPSC) could be enhanced through ADCC effect. Rituximab is anti-CD20 rituximab. X-axis, E:T ratio (RIC cells: tumor cells). Y-axis, tumor cells killing percentage. Detection was conducted after 4 hours of co-incubation.
Fig. 40 shows the *in vivo* killing of A1847 ovarian cancer tumor cells by RIC (derived from iPSC). a: Treatment flow and detection diagram. b to c: Dynamic *in vivo* imaging detection of tumor burden in the RIC treatment group and the tumor burden control group. *n*=5, statistical methods: one-way ANOVA and Kruskal-Wallis test. NS, not significant. d: Survival curve analysis. *n*=5, statistical method: Log-rank test. e: Body weight of tumor-bearing mice in the treatment group and the control group on Day 0 and Day 35. n=5, statistical method: two-sided independent t-test.;
Fig. 41 shows the expression rate of CD19-CAR of RIC cells by infection. Compared with the primary immune cells, the expression rate of CD19-CAR of RIC cells after infection with virus (MOI=20) carrying CD19-CAR was as high as 83.0%, whereas the expression rate of the natural immune cells (MOI=35) was only 33.4%;
Fig. 42 shows that CD19-CAR-iPSC-RIC killed primary tumor cells of ALL acute lymphoblastic lymphoma patients efficiently. The RIC cells carrying CD19-CAR were co-incubated *in vitro* with primary tumor cells (CD19+B cells) derived from ALL patients at a ratio of 1:1 for 6 hours, and then the number of CD19+B cells was detected. Y-axis, percentage of the residual tumor cells accounting for the initial input tumor cells.

### Specific Models for Carrying Out the present invention

The technical solutions in the embodiments of the present invention will be clearly and completely described below with reference to the accompanying drawings and the examples of the present invention. Obviously, the described examples are only some of the examples of the present invention, rather than all the examples. The following description of at least one exemplary example is merely illustrative in nature and is in no way intended to limit the present invention, its application or uses. Based on the examples of the present invention, all other examples obtained by those of ordinary skill in the art without making creative efforts fall within the scope of protection of the present invention.

Experimental methods that did not indicate specific conditions in the following examples should be selected according to conventional methods and conditions, or according to product specifications. The reagents and raw materials used in the present invention were all commercially available.

PSC was induced to differentiate into RIC with reference to Fig. 1, wherein the meaning of each symbol in Fig. 1 and the composition of culture medium were as follows:
PSC comprised ESC and iPSC; PS referred to primitive streak; LM referred to lateral plate mesoderm; hematopoietic seed cells, namely hematopoietic progenitor cells; RIC referred to induced RIC cells.
Medium I: Essential 6 medium (Gibco, A1516401 or STEMCELL Technologies, 05946) supplemented with 40 ng/mL BMP4 (R&D Systems, 314-BP-010), 30 ng/mL ACTIVIN A (PeproTech, 120-14P), 20 ng/mL bFGF (PeproTech, 100-18B), 6 µM CHIR-99021 (Selleck, S1263) and 100 nM PIK-90 (Selleck, S1187).
Medium II: Essential 6 medium supplemented with 30 ng/mL BMP4 (R&D Systems, 314-BP-010), 1 µM A-83-01 (Selleck, S7692) and 1 µM C59 (Selleck, S7037).
Medium III: Essential 6 medium supplemented with 10 µM SB431542 (selleck, S1067), 10 µM hydrocortisone (selleck, S1696), 5 ng/mL Flt3L (PeproTech, 300-19), 5 ng/mL TPO (PeproTech, 300-18), 50 ng/mL SCF (PeproTech, 300-07), 50 ng/mL EGF (PeproTech, 100-47), 50 ng/mL VEGF (R&D System,293-VE-010), 50 ng/mL bFGF (PeproTech, 100-18B), 50 ng/mL IGF-1 (PeproTech, 100-11), 50 µg/mL ascorbic acid, and 2% SUPERGROW cell culture supplement (Dakewe, 6122011).
Medium IV: 55% DMEM-high glucose (Hyclone, SH30022.01B), 30% DMEM/F12 (Hyclone, SH30023.01B), 15% SUPERGROW cell culture supplement (Dakewe, 6122011), 2 mM GlutaMAX^{™} (Gibco, 35050061), 1 µM β-mercaptoethanol (Sigma-Aldrich, M3148), 5 ng/mL sodium selenite (Sigma-Aldrich, S5261), 50 uM ethanolamine (Sigma-Aldrich, 15014), 20 µg/ mL ascorbic acid (Sigma-Aldrich, A-5960), 1% penicillin-streptomycin solution (Hyclone, SV30010), 5 ng/mL IL-3 (PeproTech, 200-03), 20 ng/mL SCF, 20 ng/ mL IL-7 (PeproTech, 200-07), 10 ng/mL IL-15 (PeproTech, 200-15) and 10 ng/mL Flt3L (PeproTech, 300-19).

The specific models for carrying out the present invention were as follows:

### Example 1: Differentiation of pluripotent stem cells (derived from ESC) into lateral plate mesoderm.

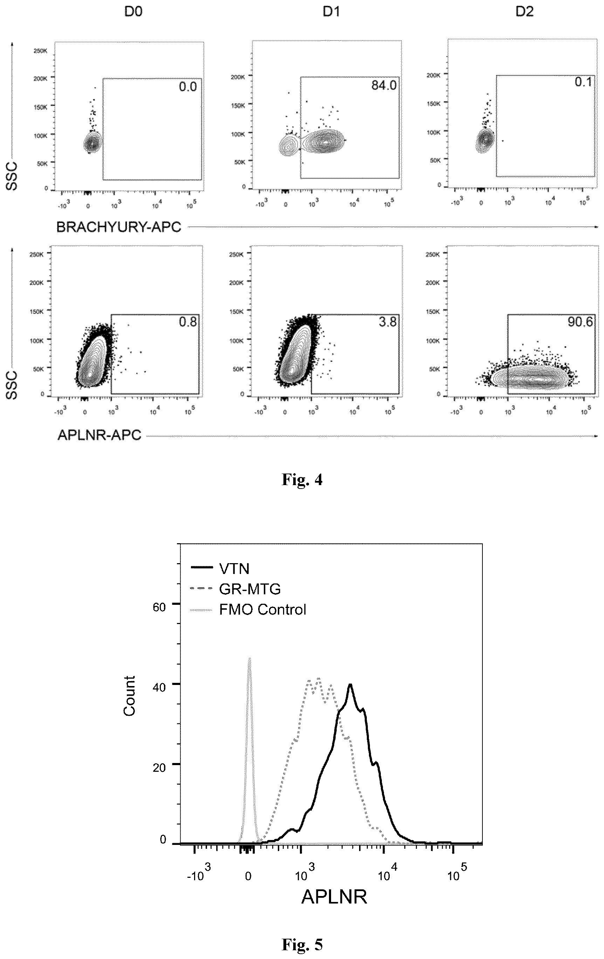

The pluripotent stem cells (derived from ESC) cultured in monolayer to 80% adherent density were digested with Accutase (Sigma-Aldrich, A6964) for 1 min, resuspended in ncTarget medium (Zhongsheng Suyuan, RP01020) into a single cell suspension, added with 2 µM ROCK inhibitor thiazovivin (Selleck, S1459), then transferred to a cell culture dish covered with VTN (Animal-Free Human Vitronectin Matrix, PeproTech, AF-VMB-220), and cultured in an incubator for 24 hours (this time was defined as Day 0 of the induction program). The ncTarget Medium in the culture dish was discarded by pipetting, and Medium I was added, and the culturing was continued in the incubator for 24 hours (this time was defined as Day 1 of the induction program). Medium I was discarded by pipetting, Medium II was added, and the culturing was continued for 24 hours (this time was defined as Day 2 of the induction program). The cells were photographed at Day 0, Day 1 and Day 2 respectively (Fig. 2), qPCR was used to detect the identity markers of the primitive streak (BRACHYURY+, its encoding gene was *TEXT*) and lateral plate mesoderm (*APLNR*+*HAND1*+*MSGN1-OSR1*-) (Fig. 3), and flow cytometry was used to detect the efficiency of induction into primitive streak and lateral plate mesoderm (Fig. 4). The reason for choosing VTN was that, compared with GR-MTG (Growth Factor Reduced Matrigel), VTN was more efficient in obtaining lateral plate mesoderm when other conditions remained unchanged (Fig. 5).

### Example 2: Culture of RIC capsule - hematopoietic progenitor cells (derived from ESC)-stage induction

The specific steps were as follows:
The lateral plate mesoderm cells were digested and prepared into a single-cell suspension, and mixed with OP9-DLL1/DLL4 trophoblast cells, the mixing ratio of the two cells could be in the range of 1:5 to 1:200, and the total number of cells was adjusted to 0.2 to 10 million according to the pore size of the culture dish to prepare a RIC capsule. Each RIC capsule occupied a culture surface area of 1 to 10 cm². Induction was continued for 10 to 18 days in Medium III, the medium was changed every 1 to 3 days, and the morphological changes of the RIC capsule unit were recorded by photographing on Day 9 and Day 16 (Fig. 6). The RIC capsule on Day 16 was selected for single-cell flow cytometry to detect hematopoietic progenitor cells (CD45+CD34+CD43+CD235a-), and this induction method was more efficient than direct monolayer cell induction of LM to obtain hematopoietic progenitor cells with CD34+ phenotype (Fig. 7).

### Example 3: Production of RIC (derived from ESC) by RIC capsule

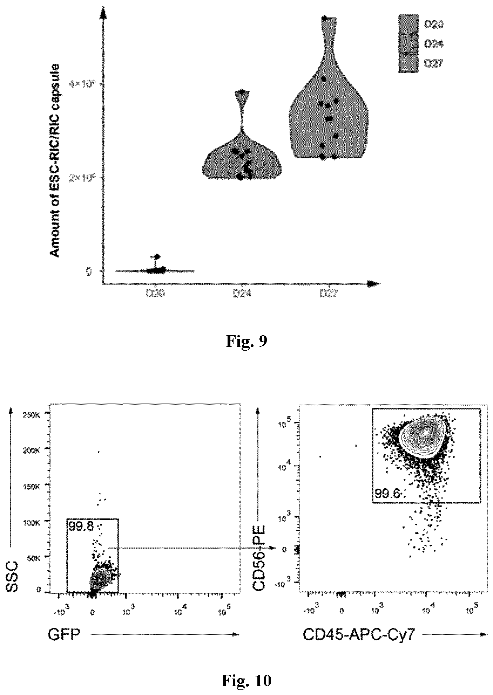

Medium III was replaced with Medium IV, and the RIC capsule was continuously cultured for 7 to 14 days, and the medium was changed every 1 to 3 days. The RIC capsule was photographed and recorded on Day 20 and Day 27 (Fig. 6). On Day 20, Day 24 and Day 27, the RIC capsule was taken to prepare single cell suspension for flow cytometry to detect ESC-RIC cells (CD45+CD56+), and sampled for statistics of cell yield (Figs 8 and 9). In addition, GFP+ trophoblast cells were no longer detectable on Day 27 (Fig. 10), and the ESC-RIC cells harvested on Day27 were photographed (Fig. 2).

### Example 4: RIC (derived from ESC) monolayer induction

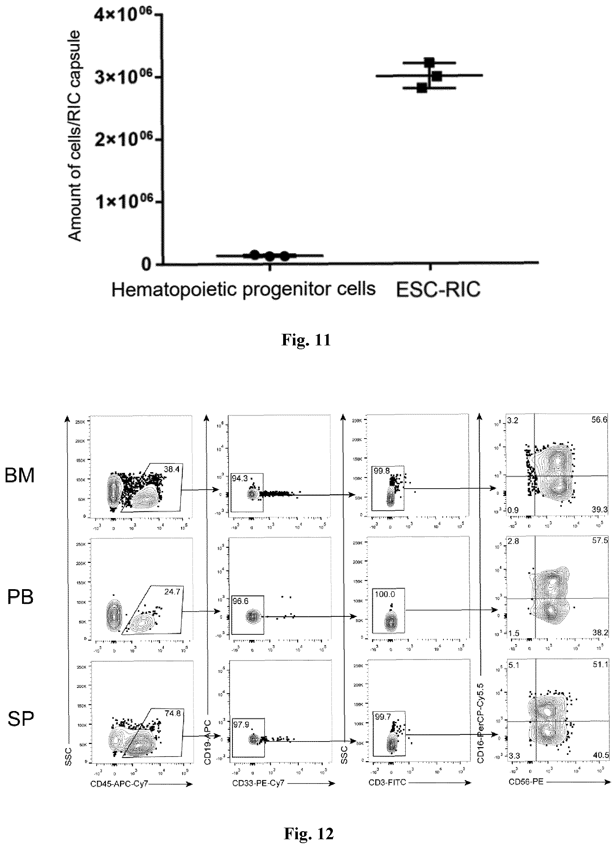

Selectively, the RIC capsule on Day 20 cultured in Medium III was taken and prepared into a single cell suspension. The GFP+ trophoblast cells were sorted out, and the remaining mixture of hematopoietic progenitor cells and immune progenitor cells was added with Medium IV and transferred to a culture dish for monolayer culturing, the medium was changed every 2 days, and the culturing was continued for 7 to 14 days for induction to obtain mature RIC cells. This monolayer induction protocol could also be used for induction to obtain RIC cells with high purity and high yield (Fig. 11).

### Example 5: In vivo induction of RIC (derived from ESC)

The hematopoietic progenitor cells in RIC capsule on Day 20 were isolated and transplanted into recipient mice. After 12 days, the detection showed that mature RIC cells were efficiently regenerated in the bone marrow (BM), peripheral blood (PB) and spleen (SP)(Fig. 12).

### Example 6: Transcriptome characteristics of RIC (derived from ESC) by single-cell sequencing.

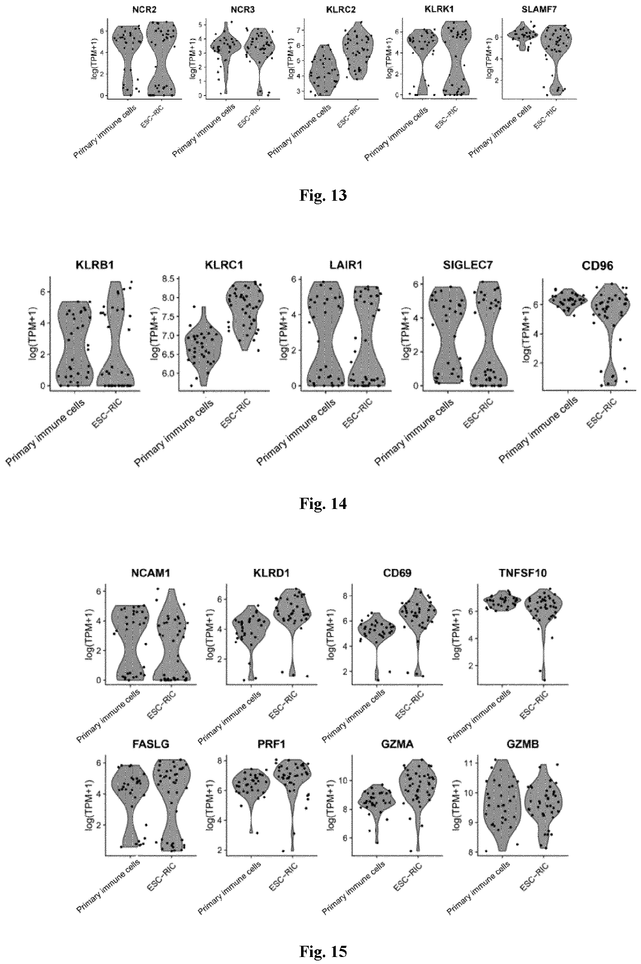

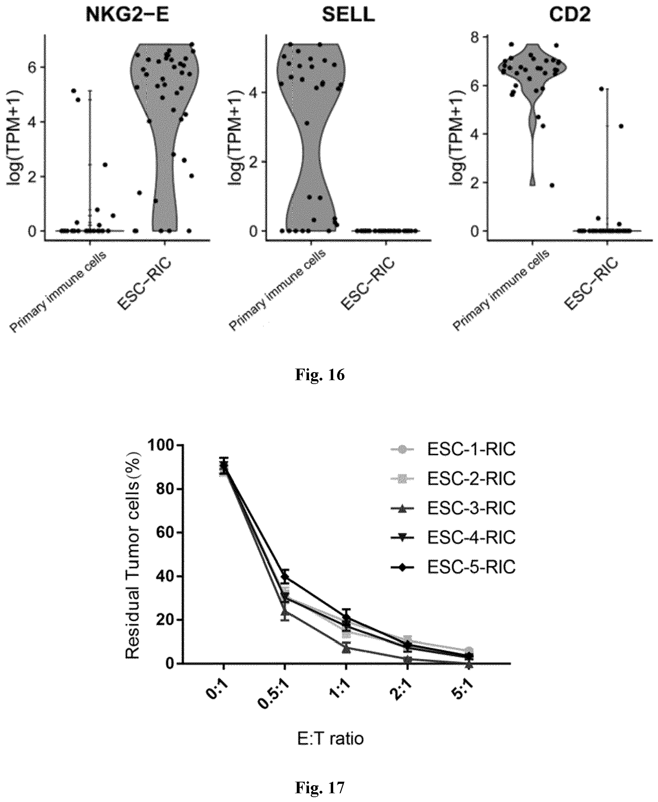

Single RIC cell and single primary immune cell derived from umbilical cord blood were subjected to deep single-cell RNA sequencing (the number of primary immune cells was 32, the number of ESC-RIC cells was 41, the sequencing method: Smartseq2). Further, the sequencing data was subjected to comparative analysis, and the result showed that, similar to primary immune cells, RIC cells expressed activating receptors NKp30, NKp44, NKG2C, NKG2D, CS1, etc. (Fig. 13), and inhibitory receptors KLRB1, KLRC1, LAIR1, SIGLEC7, CD96 etc. (Fig. 14), surface markers and functional activity-related proteins CD56, CD94, CD69, TRAIL, FasL, Perforin, Granzyme A, and Granzyme B, etc. (Fig. 15). However, RIC cells were significantly different from the natural primary immune cells, which was reflected in the unique gene expression characteristics of RIC. For example, compared with the natural primary immune cells, RIC cells had higher expression of NKG2-E and lower expression of SELL and CD2 (Fig. 16).

### Example 7: In vitro killing of Nalm-6 tumor cells by RIC (derived from ESC)

RIC cells (derived from ESC) were co-incubated with the Nalm-6 tumor cell line to test the tumor cell natural killing activity of the RIC cells. Fig. 17 showed: the RIC cells derived from pluripotent stem cells (derived from ESC) could kill tumor cells derived from lymphoma cell line Nalm-6 *in vitro.* ESC-1, -2, -3, -4, -5: five strains of ESC cell clones derived from China National Stem Cell Resource Center. X-axis, E:T ratio (RIC effector cells: tumor cells). Y-axis, percentage of the residual tumor cells. The absolute count of viable cells was detected after 6 hours of incubation.

### Example 8: In vitro efficient killing of acute myeloid leukemia (AML) cells by RIC (derived from ESC)

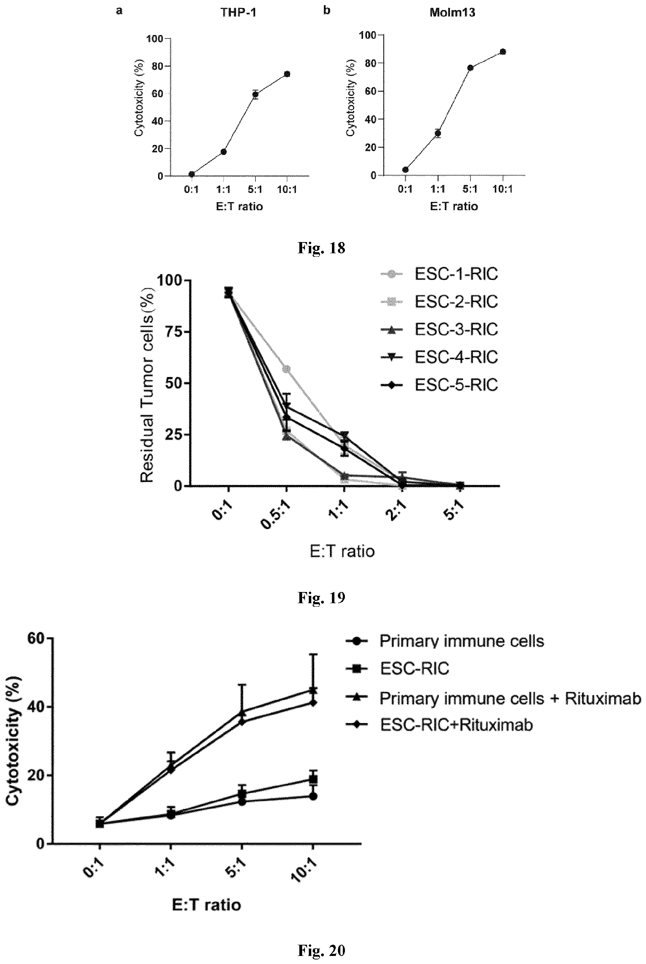

By specifically referring to Fig. 18, the RIC cells of pluripotent stem cells (derived from ESC) could kill AML cell lines THP-1 and Molm13 *in vitro.* X-axis, E:T ratio (RIC effector cells: tumor cells). Y-axis, tumor cells killing percentage. Detection was conducted after 4 hours of co-incubation.

### Example 9: In vitro killing of A1847 ovarian cancer tumor cells by RIC (derived from ESC)

By specifically referring to Fig. 19, the RIC cells derived from pluripotent stem cells (derived from ESC) could kill tumor cells derived from ovarian cancer cell line A1847 *in vitro.* ESC-1, -2, -3, -4, -5: five strains of ESC cell clones derived from National Stem Cell Resource Center. X-axis, E:T ratio (RIC cells: tumor cells). Y-axis, percentage of the residual tumor cells accounting for the initial input tumor cells. Detection was conducted after 4 hours of co-incubation.

### Example 10: Mediation and enhancement of anti-tumor activity through ADCC effect by RIC (derived from ESC) in combination with monoclonal antibody

By specifically referring to Fig. 20, the killing of B lymphoma cell line Raji by RIC cells (derived from ESC) could be enhanced through ADCC effect. Rituximab was anti-CD20 rituximab. X-axis, E:T ratio (RIC cells: tumor cells). Y-axis, tumor cells killing percentage. Detection was conducted after 4 hours of co-incubation.

### Example 11: In vivo killing of A1847 ovarian cancer tumor cells by RIC (derived from ESC)

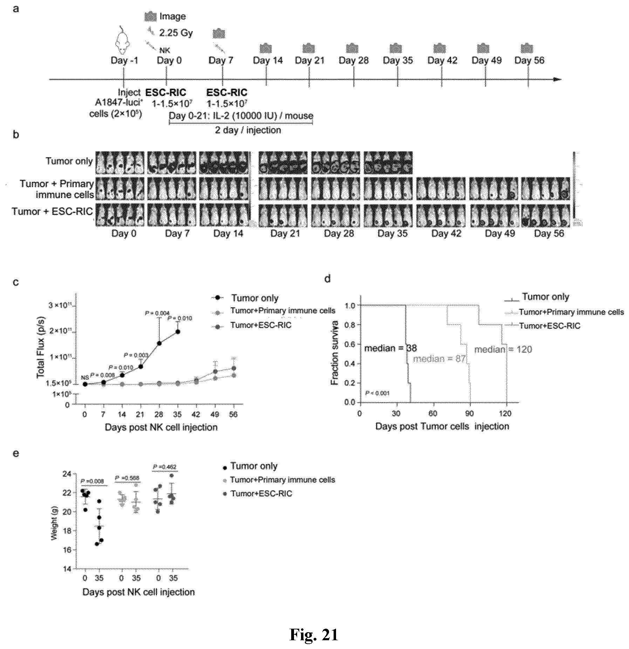

First, the A1847 ovarian cancer tumor mouse model was established. Each of 6 to 8-week-old NSG mice (immune deficient mice without RIC, T, B immune cells) was inoculated with 2×10⁵ A1847-luciferase+ (luciferase positive) cells by intraperitoneal injection. One day after tumor cell inoculation, the tumor model was subjected to living imaging (IVIS Spectrum, PerkinElmer). Then, each mouse was transplanted with 1 to 1.5 ×10⁷ RIC cells (500 µL) by intraperitoneal injection to perform treatment. Infusion was carried out once per week, and continued for two weeks. IL-2 (10000 U/mouse) was injected within 21 days after the treatment, once every two days. The mice were subjected to living imaging every 3 to 4 days to analyze the changes of tumor burden. ESC-RIC could effectively kill A1847 ovarian cancer cells *in vivo* (Fig. 21).

### Example 12: In vivo killing of AML cells by RIC (derived from ESC)

First, the HL60 AML mouse model was established. Each of 6 to 8-week-old NSG mice (immune deficient mice without RIC, T, B immune cells) was inoculated with 1×10⁶ HL60-luciferase+ (luciferase positive) cells by intraperitoneal injection. Three days after tumor cell inoculation, the tumor model was subjected to living imaging (IVIS Spectrum, PerkinElmer). Then, each mouse was transplanted with 1 to 1.5 ×10⁷ RIC cells (200 µL) via tail vein injection to perform treatment. Infusion was carried out three times per week, and continued for 2 to 3 weeks. The mice were subjected to living imaging every 7 days to analyze the changes of tumor burden. ESC-RIC could effectively kill HL60 AML cells (Fig. 22).

### Example 13: Killing of primary tumor cells of ALL acute lymphoblastic leukemia patients by CD19-CAR-ESC-RIC (derived from ESC)

First, CD19-CAR lentivirus was used to infect cord blood-derived primary immune cells and ESC-RIC cells. 5×10⁶ primary immune cells and ESC-RIC cells were each infected with CD19-CAR lentivirus by centrifugal infection (primary immune cells: MOI=35, ESC-RIC cells: MOI=20). After the cells were infected for 48 hours, flow cytometry was used to detect the infection efficiency. First, a small amount of cells (2×10⁵ for each kind of cells) were taken and incubated with CD56-APC and Anti-Mouse FMC63 scFv Monoclonal Antibody, PE (CD19-CAR-PE ) antibody; after the incubation, flow cytometry was used to detect the infection efficiency, wherein the uninfected primary immune cells (NC) and ESC-RIC (NC) were used as negative controls to detect the positive rate of CD19-CAR. The infection efficiency of CD19-CAR-primary immune cells was 14.9%, and that of CD19-CAR-ESC-RIC was as high as 82.4% (Fig. 23). The RIC after expression of CD19-CAR could efficiently kill the B cells in patients with acute lymphoblastic leukemia *in vitro* (Fig. 24). Fig. 24: CD19-CAR-ESC-RIC could efficiently kill primary tumor cells in ALL acute lymphoblastic leukemia patients. The RIC cells carrying CD19-CAR were co-incubated *in vitro* with primary tumor cells (CD19+B cells) derived from two ALL patients at a ratio of 1:1 for 6 hours, and then the number of CD19+B cells was detected. Y-axis, percentage of the residual tumor cells accounting for the initial input tumor cells.

### Example 14: Differentiation of pluripotent stem cells (derived from iPSC) into lateral plate mesoderm

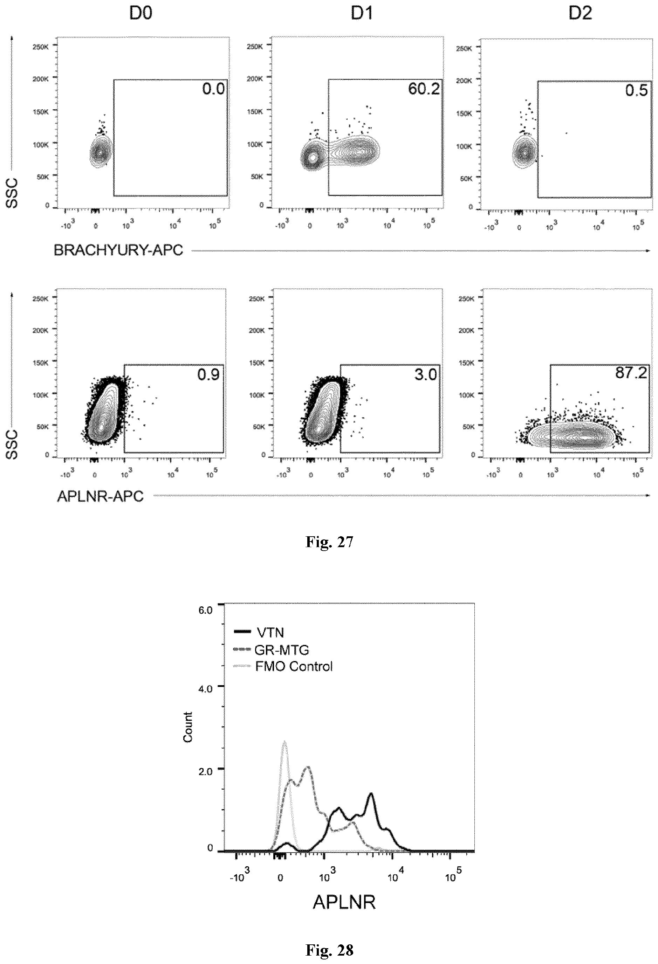

Pluripotent stem cells (derived from iPSCs) cultured in monolayer to 80% adherent density were digested with Accutase (Sigma-Aldrich, A6964) for 1 min, and resuspended in ncTarget medium (Zhongsheng Suyuan, RP01020) to obtain a single cell suspension, added with 2 µM ROCK inhibitor thiazovivin (Selleck, S 1459), then transferred to a cell culture dish covered with VTN (Animal-Free Human Vitronectin Matrix, PeproTech, AF-VMB-220), and cultured in an incubator for 24 hours (this time was defined as Day 0 of the induction program). The ncTarget Medium in the culture dish was discarded by pipetting, Medium I was added, and the culturing was continued in the incubator for 24 hours (this time was defined as Day 1 of the induction program). Medium I was discarded by pipetting, Medium II was added, and the culturing was continued for 24 hours (this time was defined as Day 2 of the induction program). The cells were photographed at Day 0, Day 1 and Day 2 respectively (Fig. 25), and qPCR was used to detect the identity markers of the primitive streak (BRACHYURY+, its encoding gene was *TBXT*) and lateral plate mesoderm (*APLNR+HAND1+MSGN1-OSR1-*) (Fig. 26), and flow cytometry was used to detect the efficiency into primitive streak and lateral plate mesoderm (Fig. 27). The reason for choosing VTN was that, compared with GR-MTG (Growth Factor Reduced Matrigel), VTN was more efficient in obtaining lateral plate mesoderm when other conditions remained unchanged (Fig. 28).

### Example 15: Production of RIC by RIC capsule (derived from iPSC)

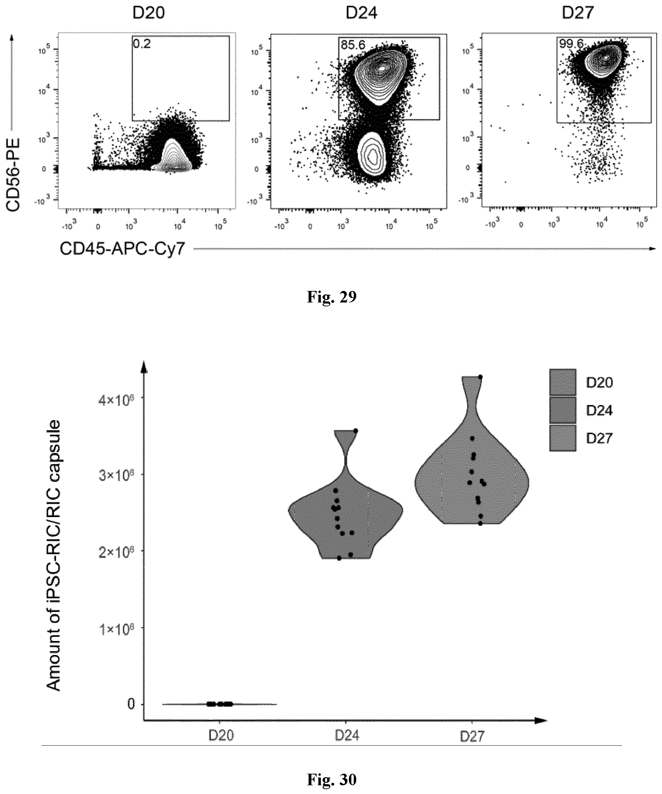

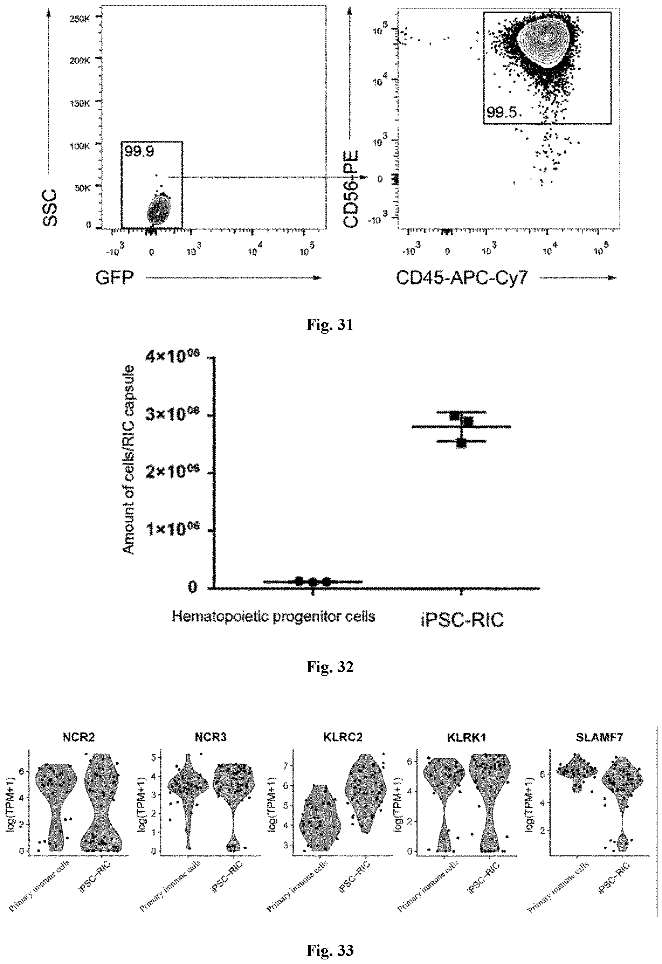

Referring to the methods in Examples 1 to 4, the iPSC-derived RIC cells could be obtained under the induction of Media I, II, III, and IV. The RIC capsules were taken on Day 20, Day 24 and Day 27, respectively, to prepare single cell suspensions for flow cytometry to detect iPSC-RIC cells (CD45+CD56+), and sampled for the statistics of cell yield (Figs 29 and 30). In addition, GFP+ trophoblast cells were no longer detectable on Day 27 (Fig. 31).

### Example 16: RIC (derived from iPSC) monolayer induction

Selectively, the RIC capsules on Day 20 cultured in Medium III were taken and prepared into a single cell suspension. The GFP+ trophoblast cells were sorted out. The remaining mixture of hematopoietic progenitor cells and immune progenitor cells was added with Medium IV and transferred to a culture dish for monolayer culturing, the medium was changed every 2 days, and the culturing was continued for 7 to 14 days for induction to obtain mature RIC cells. This monolayer induction protocol could also be used for induction to obtain RIC cells with high purity and high yield (Fig. 32).

### Example 17: Transcriptome characteristics of RIC (derived from iPSC) by single-cell sequencing.

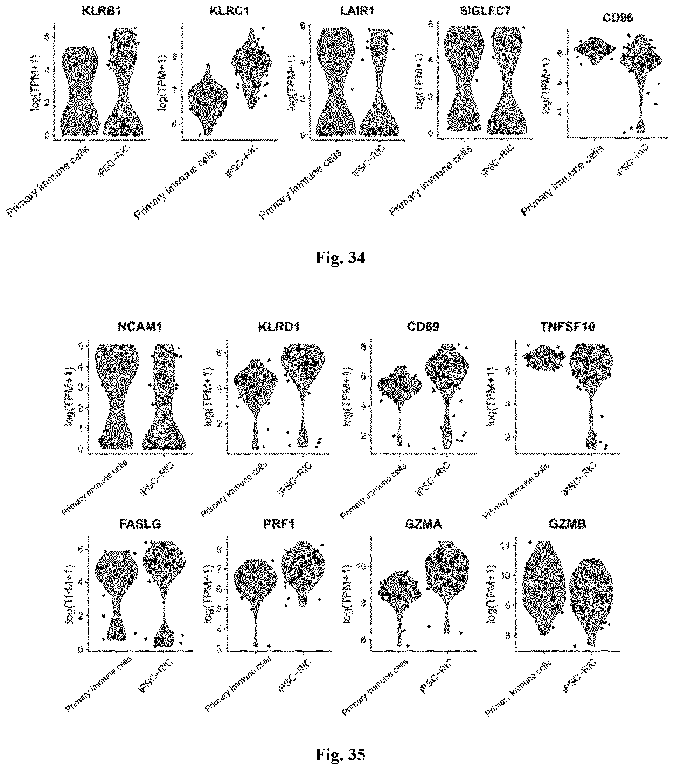

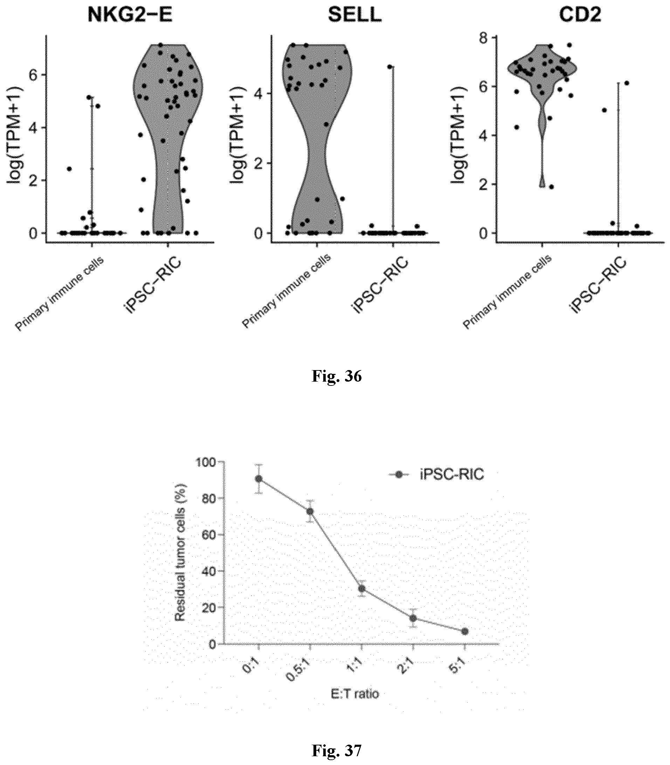

Single RIC cell and single primary immune cell derived from umbilical cord blood were subjected to deep single-cell RNA sequencing (the number of primary immune cells was 32, the number of iPSC-RIC cells was 41, the sequencing method: Smartseq2). Further, the sequencing data was subjected to comparative analysis, and the result showed that, similar to the primary immune cells, RIC cells expressed activating receptors NKp30, NKp44, NKG2C, NKG2D, CS1, etc. (Fig. 33), inhibitory receptors KLRB1, KLRC1, LAIR1, SIGLEC7, CD96 (Fig. 34), surface markers and functional activity-related proteins CD56, CD94, CD69, TRAIL, FasL, Perforin, Granzyme A, and Granzyme B, etc. (Fig. 35). However, RIC was significantly different from the natural RIC, which was reflected in the unique gene expression characteristics of RIC. For example, compared with the natural primary immune cells, RIC had higher expression of NKG2-E and lower expression of SELL and CD2 (Fig. 36).

### Example 18: In vitro killing of Nalm-6 tumor cells by RIC (derived from iPSC)

RIC (derived from iPSCs) was co-incubated with Nalm-6 tumor cell line to detect the tumor cell natural killing activity of RIC. Fig. 37 showed: RIC cells derived from pluripotent stem cells (derived from iPSC) killed the tumor cells derived from lymphoma cell line Nalm-6 *in vitro.* X-axis, E:T ratio (RIC effector cells: tumor cells). Y-axis, percentage of the residual tumor cells. The absolute count of viable cells was detected after 6 hours of incubation.

### Example 19: In vitro killing of A1847 ovarian cancer tumor cells by RIC (derived from iPSC)

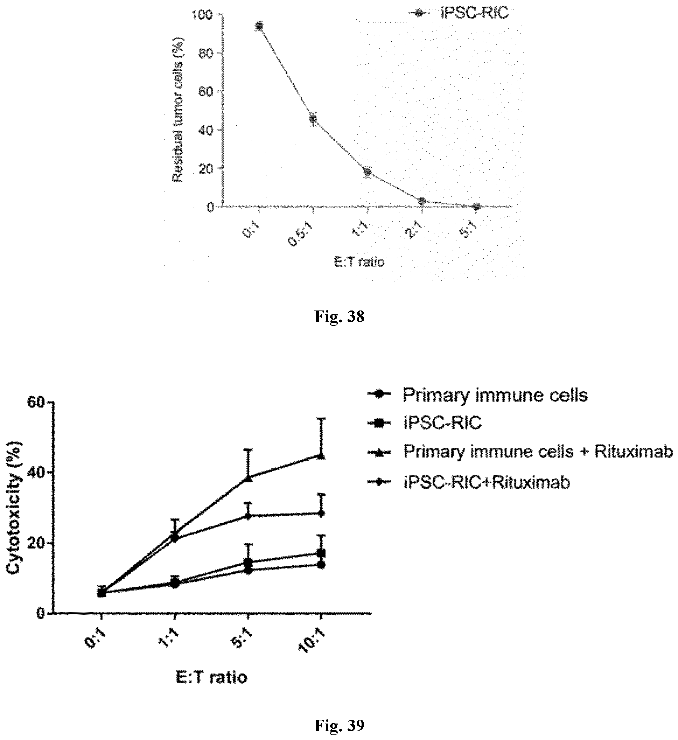

Fig. 38 showed: the RIC cells derived from pluripotent stem cells (derived from iPSC) killed the tumor cells derived from the ovarian cancer cell line A1847 *in vitro.* X-axis, E:T ratio (RIC cells: tumor cells). Y-axis, percentage of the residual tumor cells accounting for the initial input tumor cells. Detection was conducted after 4 hours of co-incubation.

### Example 20: Mediation and enhancement of anti-tumor activity through ADCC effect by RIC (derived from iPSC) in combination with monoclonal antibody

By specifically referring to Fig. 39: the killing of B lymphoma cell line Raji by the iPSC-RIC cells could be enhanced through ADCC effect. Rituximab was anti-CD20 rituximab. X-axis, E:T ratio (RIC cells: tumor cells). Y-axis, tumor cells killing percentage. Detection was conducted after 4 hours of co-incubation.

### Example 21: In vivo killing of A1847 ovarian tumor cells by RIC (derived from iPSC)

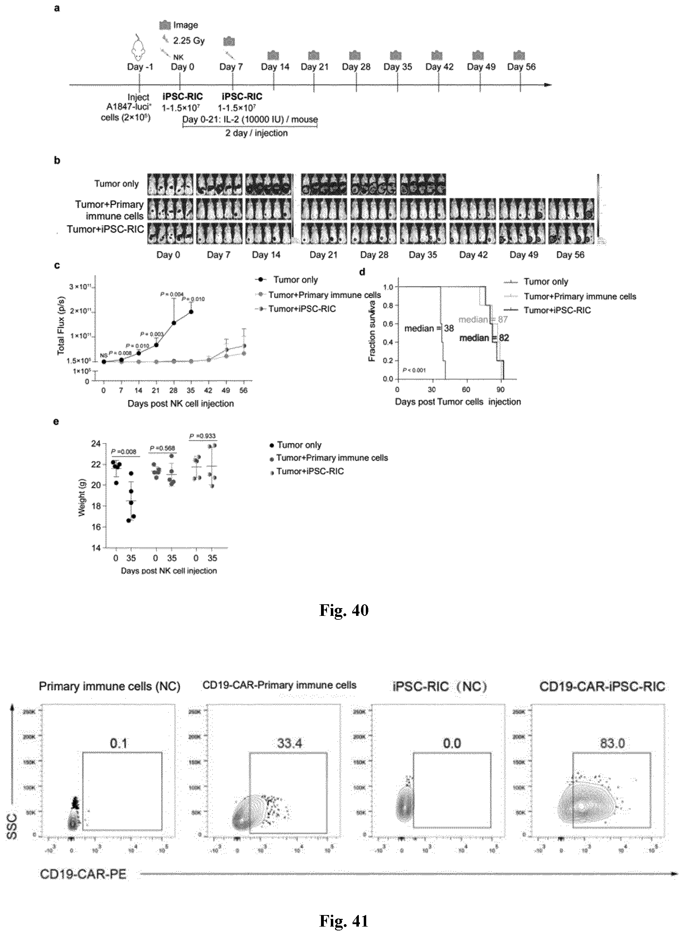

First, the A1847 ovarian tumor mouse model was established. Each of 6 to 8-week-old NSG mice (immune deficient mice without RIC, T, B immune cells) was inoculated with 2×10⁵ A1847-luciferase+ (luciferase positive) cells by intraperitoneal injection. One day after tumor cell inoculation, the tumor model was subjected to living imaging (IVIS Spectrum, PerkinElmer). Then, each of the mice was transplanted with 1 to 1.5 ×10⁷ RIC cells (500 µL) by intraperitoneal injection to perform treatment. Infusion was carried out once per week, and continued for two weeks. IL-2 (10000 U/mouse) was injected within 21 days after the treatment, once every two days. The mice were subjected to living imaging every 3 to 4 days to analyze changes of tumor burden. The iPSC-RIC could effectively kill A1847 ovarian tumor cells *in vivo.* Fig. 40 showed: RIC (derived from iPSC) killed A1847 ovarian tumor cells *in vivo,* wherein, A showed schematic diagram of treatment flow and detection, B showed dynamic living imaging detection of tumor burden in the RIC treatment group and the tumor burden control group.

### Example 22: Killing of primary tumor cells of ALL acute lymphoblastic leukemia patients by CD19-CAR-iPSC-RIC (derived from iPSC)

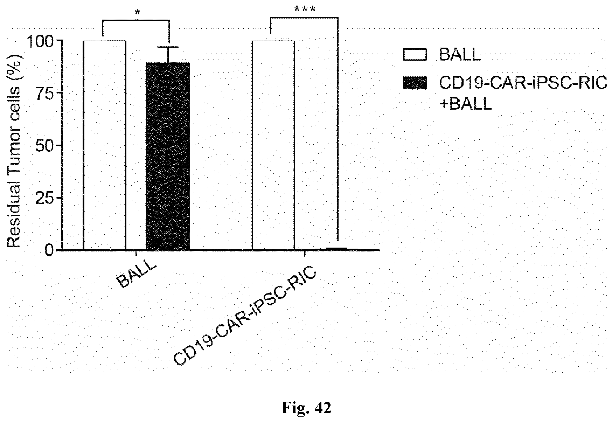

First, CD19-CAR lentivirus was used to infect primary immune cells derived from cord blood and iPSC-RIC cells. 5×10⁶ primary immune cells and iPSC-RIC cells were each infected with CD19-CAR lentivirus by centrifugal infection (primary immune cells: MOI=35, iPSC-RIC cells: MOI=20). After the cells were infected for 48 hours, flow cytometry was used to detect the infection efficiency. First, a small amount of cells (2×10⁵ for each kind of cells) were taken and incubated with CD56-APC and Anti-Mouse FMC63 scFv Monoclonal Antibody, PE (CD19-CAR-PE ) antibody; after the incubation, flow cytometry was used to detect the transduction efficiency, the uninfected primary immune cells (NC) and iPSC-RIC (NC) were used as negative controls to detect the positive rate of CD19-CAR. Fig. 41 showed the infection rate of RIC cells introduced with CD19-CAR by infection. Compared with the primary immune cells, the expression rate of RIC cells after infection with CD19-CAR virus (MOI=20) was as high as 83.0%, whereas the expression rate of the natural immune cells (MOI=35) was only 33.4%. Fig. 42 showed that CD19-CAR-iPSC-RIC efficiently killed the primary tumor cells of ALL acute lymphoblastic leukemia patients. The RIC cells carrying CD19-CAR were co-incubated *in vitro* with the primary tumor cells (CD19+B cells) derived from ALL patients at a ratio of 1:1 for 6 hours, and then the number of CD19+B cells was detected. Y-axis, percentage of residual tumor cells accounting for the initial input tumor cells.

Various modifications of the present invention in addition to those described herein will become apparent to those skilled in the art from the foregoing description, and such modifications are intended to fall within the scope of the appended claims.

## Claims

**1.** A population of RIC cells, wherein the average expression level of NKG2-E of the population of RIC cells is at least about 10 times that of primary immune cells.

**2.** The population of RIC cells according to claim 1, wherein the expression level of SELL and CD2 of the population of RIC cells is at least about 10 times less than that of primary immune cells.

**3.** The population of RIC cells according to claim 1 or 2, wherein the population of RIC cells is introduced with a target CAR by a gene delivery vector or continuously or conditionally expresses a target CAR by gene editing, and the expression rate of CAR is 60% to 100%.

**4.** The population of RIC cells according to any one of claims 1 to 3, wherein the population of RIC cells also has the following characteristics:
(1) having any three or more of the activating receptors NCR1, NCR2, NCR3, KLRC2, KLRK1 and SLAMF7; and/or
(2) having any three or more of the inhibitory receptors KLRB1, KLRC1, LAIR1, SIGLEC7 and CD96.

**5.** The population of RIC cells according to any one of claims 1 to 4, which further has the following characteristic: ≥80% cells express both CD45 and CD56.

**6.** The population of RIC cells according to any one of claims 1 to 5, wherein the population of RIC cells is generated from stem cells;
preferably, the stem cells are totipotent stem cells or pluripotent stem cells, further preferably, the stem cells are pluripotent stem cells, further preferably, the pluripotent stem cells are selected from the group consisting of embryonic stem cells, haploid stem cells and induced pluripotent stem cells.

**7.** A method for producing the population of RIC cells according to any one of claims 1 to 6, which comprises the following steps:
(1) culturing stem cells to form lateral plate mesoderm cells;
(2) inducing the lateral plate mesoderm cells to differentiate into hematopoietic progenitor cells;
(3) inducing the hematopoietic progenitor cells to differentiate into the RIC cells.

**8.** The method according to claim 7, wherein step (2) comprises: preparing a RIC capsule and cultivating the RIC capsule in a culture medium, the RIC capsule further has the following characteristics: it is composed of the lateral plate mesoderm cells derived from step (1) and trophoblast cells, and the lateral plate mesoderm cells are mixed with the trophoblast cells in a ratio ranging from 1:5 to 1:200.

**9.** The method according to claim 8, wherein the trophoblast cells of the RIC capsule are one or more selected from AFT024, OP9, MS5, HS-5 and other stromal cells, and preferably express at least one, two or three of the following genes: DLL1, DLL4, IL7, IL15, IL3 and Flt3L.

**10.** The method according to any one of claims 8 to 9, wherein the total number of cells of a single RIC capsule is in the range of 0.2 to 10 million cells, and the culture area occupied by each RIC capsule is in the range of 1 to 10 cm², preferably the RIC capsule is continuously cultured under the medium conditions of step (2) for 10 to 18 days, and then continuously cultured under the medium conditions of step (3) for 7 to 14 days.

**12.** The method according to any one of claims 7 to 11, wherein, culturing the stem cells to form lateral plate mesoderm cells in step (1) by using Medium I, and Medium I is a basic medium supplemented with the following substances: one or more serum substitutes, one or more non-essential amino acids, glutamine or a stabilized dipeptide of L-alanyl-L-glutamine, as well as BMP4, ACTIVIN, bFGF, Wnt agonist and PI3K inhibitor.

**13.** The method according to any one of claims 7 to 12, wherein, culturing the stem cells to form lateral plate mesoderm cells in step (1) by further adding Medium II, and Medium II is a basic medium supplemented with the following substances: one or more serum substitutes, one or more non-essential amino acids, glutamine or a stabilized dipeptide of L-alanyl-L-glutamine, as well as BMP4, TGF-β inhibitor and Wnt inhibitor.

**14.** The method according to any one of claims 7 to 13, wherein, inducing the lateral plate mesoderm cells for differentiation into hematopoietic progenitor cells in step (2) by using Medium III, and Medium III is a basic culture medium supplemented with the following substances: one or more serum substitutes, one or more non-essential amino acids, glutamine or a stabilized dipeptide of L-alanyl-L-glutamine, as well as a cytokine (e.g., one, two or more selected from the group consisting of Flt3L, TPO, SCF, EGF, VEGF, bFGF and IGF-1).

**15.** The method according to any one of claims 7-14, wherein, inducing the hematopoietic progenitor cells for differentiation into RIC cells in step (3) by using Medium IV, and Medium IV is a basic culture medium supplemented with the following substances: one or more serum substitutes, one or more non-essential amino acids, glutamine or a stabilized dipeptide of L-alanyl-L-glutamine, as well as a cell growth factor (e.g., one, two or more selected from the group consisting of IL-3, SCF, IL-7, IL-15 and Flt3L).

**16.** The method according to claim 14, wherein the Medium IV further comprises β-mercaptoethanol; preferably, the content of β-mercaptoethanol is 0.1% to 0.5% (v/v).

**17.** The method according to any one of claims 12-16, wherein the serum substitute is one or more selected from the group consisting of KOSR, B27, serum-free additive, Ultroser^{™} G, and SUPERGROW in any combination.

**18.** The method according to any one of claims 7 to 17, wherein step (1) lasts 1 to 3 days for culture, step (2) lasts 10 to 18 days for culture, and step (3) lasts 7 to 14 days for culture.

**19.** The method according to any one of claims 7 to 18, wherein, conducting the culture of step (1) in a matrix, and the matrix is one or more selected from the group consisting of glycoprotein (e.g., vitronectin (VTN), fibronectin), hyaluronic acid, laminin, fibronectin, collagen, elastin, heparan sulfate, dextran, dextran sulfate, and chondroitin sulfate.

**20.** A RIC capsule, which has the characteristics of the RIC capsule according to any one of claims 8 to 10.

**21.** An intermediate cell combination, comprising lateral plate mesoderm cells formed by culturing stem cells, such as the lateral plate mesoderm cells produced by step (1) of the method according to any one of claims 7 to 19;
preferably, more than 65% of the lateral plate mesoderm cells are APLNR+ cells, and further preferably, more than 65% are APLNR+HAND1+ cells.

**22.** An intermediate cell combination, comprising hematopoietic progenitor cells produced in step (2) of the method according to any one of claims 7 to 19 and other immune progenitor cells (e.g., myeloid cells);
preferably, more than 65% of the hematopoietic progenitor cells are CD45+CD43+CD34+CD235a- cells.

**23.** A modified population of RIC cells, which is a population of CAR-RIC cells formed from the population of RIC cells according to any one of claims 1 to 6 that expresses a chimeric antigen receptor (CAR).

**24.** A culture, which comprises the population of RIC cells according to any one of claims 1 to 6, and a culture medium.

**25.** A culture supernatant, which is a culture supernatant produced by culturing the population of RIC cells according to any one of claims 1 to 6 in a culture medium.

**26.** A composition, which comprises the population of RIC cells according to any one of claims 1 to 6, the culture according to claim 24, or the culture supernatant according to claim 25, and a carrier or excipient;
preferably, the composition is an injection, a microinjection, a mucosal patch, an enema, a suppository, a gel, an oral agent, an aerosol, a drop, an ointment, an implant, a capsule or an aerosol;
preferably, the composition is an injection;
preferably, the composition comprises a pharmaceutically acceptable sterile isotonic aqueous or non-aqueous solution, dispersion, suspension or emulsion;
preferably, the composition is a pharmaceutical composition.

**27.** A kit, which comprises one, two or more of Media I, II, III, IV, and
the Medium I, II, III and IV are as defined in any one of claims 7 to 19.

**28.** Use of the population of RIC cells according to any one of claims 1 to 6, a population of cells derived from the population of RIC cells according to any one of claims 1 to 6, the culture according to claim 24, or the culture supernatant according to claim 25, or the composition according to claim 26 in the manufacture of a medicament for preventing and/or treating a disease in a subject, wherein the disease is selected from the group consisting of cancer, infectious disease, immunodeficiency, immunocompromise, autoimmune disease or immunodegenerative disease.

**29.** The use according to claim 28, wherein the disease is selected from the group consisting of viral pneumonia, AIDS, lymphoma, leukemia, breast cancer, ovarian cancer, liver cancer, glioma, pancreatic cancer, human HIV virus, rhinovirus, cytomegalovirus CMV, hepatitis B virus HBV, herpes simplex virus HSV, herpes zoster virus.

**30.** A method for preventing and/or treating a disease, comprising a step of administering a prophylactically and/or therapeutically effective amount of the population of RIC cells according to any one of claims 1 to 6, a population of cells derived from the population of RIC cells according to any one of claims 1 to 6, the culture according to claim 24, the culture supernatant according to claim 25, or the composition according to claim 26 to a subject in need thereof, wherein the disease is selected from cancer, infectious disease, immunodeficiency, immunocompromise, autoimmune disease or immunodegenerative disease.

**31.** The method according to claim 30, wherein the disease is selected from the group consisting of viral pneumonia, AIDS, lymphoma, leukemia, breast cancer, ovarian cancer, liver cancer, glioma, pancreatic cancer, human HIV virus, rhinovirus, cytomegalovirus CMV, hepatitis B virus HBV, herpes simplex virus HSV, herpes zoster virus.
